# EUROPEAN PATENT APPLICATION

(11) **EP 3 626 724 A1**
(43) Date of publication of application: **25.03.2020**
(21) Application number: 19180796.5
(22) Date of filing: 18.06.2019
(51) Int. Cl.: C07F 15/00, C09K 11/06, H01L 51/00

(54) **ORGANOMETALLIC COMPOUND, ORGANIC LIGHT-EMITTING DEVICE INCLUDING THE SAME, AND DIAGNOSTIC COMPOSTION INCLUDING THE ORGANOMETALLIC COMPOUND**

(30) Priority: 19.09.2018 KR 20180112385
(71) Applicant: Samsung Electronics Co., Ltd., Gyeonggi-do 16677 (KR)
(72) Inventor: JEON, Aram, 16678 Gyeonggi-do (KR); KOO, Hyun, 16678 Gyeonggi-do (KR); KIM, Sangdong, 16678 Gyeonggi-do (KR); CHO, Yongsuk, 16678 Gyeonggi-do (KR); CHOI, Jongwon, 16678 Gyeonggi-do (KR); HWANG, Kyuyoung, 16678 Gyeonggi-do (KR); KWAK, Yoonhyun, 16678 Gyeonggi-do (KR); KWON, Ohyun, 16678 Gyeonggi-do (KR)
(74) Representative: Elkington and Fife LLP

(57) **Abstract**

An organometallic compound represented by Formula 1:

Formula 1 M(L₁)ₙ₁(L₂)ₙ₂

wherein, in Formula 1, M, L₁, L₂, n1, and n2 are the same as described in the specification.

## Description

### FIELD OF THE INVENTION

One or more embodiments relate to an organometallic compound, an organic light-emitting device including the organometallic compound, and a diagnostic composition including the organometallic compound.

### BACKGROUND OF THE INVENTION

Organic light-emitting devices (OLEDs) are self-emission devices, which have superior characteristics in terms of a viewing angle, a response time, a brightness, a driving voltage, and a response speed, and which produce full-color images.

In an example, an organic light-emitting device includes an anode, a cathode, and an organic layer disposed between the anode and the cathode, wherein the organic layer includes an emission layer. A hole transport region may be disposed between the anode and the emission layer, and an electron transport region may be disposed between the emission layer and the cathode. Holes provided from the anode may move toward the emission layer through the hole transport region, and electrons provided from the cathode may move toward the emission layer through the electron transport region. The holes and the electrons recombine in the emission layer to produce excitons. These excitons transit from an excited state to a ground state, thereby generating light.

Meanwhile, luminescent compounds may be used to monitor, sense, or detect a variety of biological materials including cells and proteins. An example of the luminescent compounds includes a phosphorescent luminescent compound.

Various types of organic light emitting devices are known. However, there still remains a need in OLEDs having low driving voltage, high efficiency, high brightness, and long lifespan.

### SUMMARY OF THE INVENTION

Provided are a novel organometallic compound, an organic light-emitting device including the same, and a diagnostic composition including the novel organometallic compound.

Additional aspects will be set forth in part in the description which follows and, in part, will be apparent from the description, or may be learned by practice of the presented embodiments.

According to an aspect of an embodiment, an organometallic compound is represented by Formula 1:

Formula 1 M(L₁)ₙ₁(L₂)ₙ₂.

In Formula 1,
M may be a transition metal,
L₁ may be a ligand represented by Formula 2 or Formula 3,
n1 may be 1, 2, or 3, wherein, when n1 is two or more, two or more groups L₁ may be identical to or different from each other,
L₂ may be selected from a monodentate ligand, a bidentate ligand, a tridentate ligand, and a tetradentate ligand,
n2 may be 0, 1, 2, 3, or 4, wherein, when n2 is two or more, two or more groups L₂ may be identical to or different from each other, and
L₁ and L₂ may be different from each other, 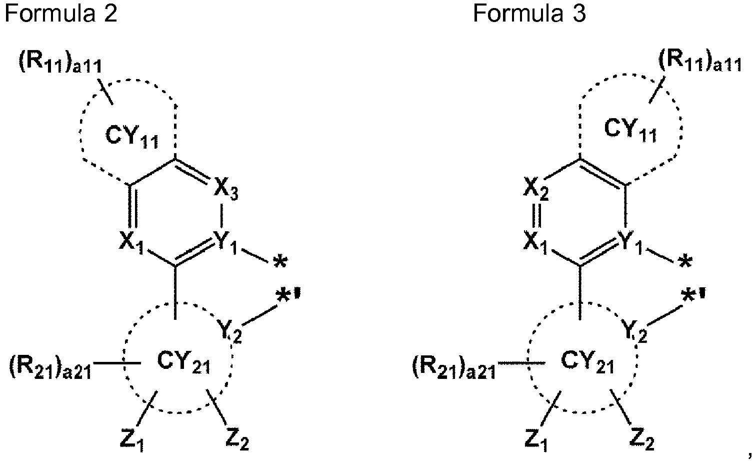
wherein, in Formulae 2 and 3,
CY₁₁ and CY₂₁ may each independently be selected from a C₅-C₃₀ carbocyclic group and a C₁-C₃₀ heterocyclic group,
Y₁ and Y₂ may each independently be selected from C and N,
X₁ may be N or C(R₁), X₂ may be N or C(R₂), X₃ may be N or C(R₃),
R₁ to R₃, R₁₁, and R₂₁ may each independently be selected from hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₇-C₆₀ arylalkyl group, a substituted or unsubstituted C₁-C₆₀ heteroaryl group, a substituted or unsubstituted C₁-C₆₀ heteroaryloxy group, a substituted or unsubstituted C₁-C₆₀ heteroarylthio group, a substituted or unsubstituted C₂-C₆₀ heteroarylalkyl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), -Ge(Q₃)(Q₄)(Q₅), -B(Q₆)(Q₇), and -P(=O)(Q₈)(Q₉),
a11 may be an integer from 0 to 10, wherein, when a11 is two or more, two or more groups R₁₁ may be identical to or different from each other,
a21 may be an integer from 0 to 8, wherein, when a21 is two or more, two or more groups R₂₁ may be identical to or different from each other,
Z₁ and Z₂ may each independently be selected from a C₁-C₆₀ alkyl group and a deuterium-containing C₁-C₆₀ alkyl group,
Z₁ and Z₂ may be different from each other,
* and *' each indicate a binding site to M in Formula 1,
at least one substituent of the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₇-C₆₀ arylalkyl group, the substituted C₁-C₆₀ heteroaryl group, the substituted C₁-C₆₀ heteroaryloxy group, the substituted C₁-C₆₀ heteroarylthio group, the substituted C₂-C₆₀ heteroarylalkyl group, the substituted monovalent non-aromatic condensed polycyclic group, and the substituted monovalent non-aromatic condensed heteropolycyclic group may be selected from:
   deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
   a C₁-C₆₀ alkyl group, a C₂-C₆₀ alkenyl group, a C₂-C₆₀ alkynyl group, and a C₁-C₆₀ alkoxy group, each substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, a monovalent non-aromatic condensed heteropolycyclic group, -N(Q₁₁)(Q₁₂),-Si(Q₁₃)(Q₁₄)(Q₁₅), -Ge(Q₁₃)(Q₁₄)(Q₁₅), -B(Q₁₆)(Q₁₇), and -P(=O)(Q₁₈)(Q₁₉);
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, and a monovalent non-aromatic condensed heteropolycyclic group;
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, and a monovalent non-aromatic condensed heteropolycyclic group, each substituted with at least one selected from deuterium, -F, -Cl, -Br, -I, -CD₃,-CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, a monovalent non-aromatic condensed heteropolycyclic group, -N(Q₂₁)(Q₂₂),-Si(Q₂₃)(Q₂₄)(Q₂₅), -Ge(Q₂₃)(Q₂₄)(Q₂₅), -B(Q₂₆)(Q₂₇), and -P(=O)(Q₂₈)(Q₂₉); and
   -N(Q₃₁)(Q₃₂), -Si(Q₃₃)(Q₃₄)(Q₃₅), -Ge(Q₃₃)(Q₃₄)(Q₃₅), -B(Q₃₆)(Q₃₇), and-P(=O)(Q₃₈)(Q₃₉), and
   Q₁ to Q₉, Q₁₁ to Q₁₉, Q₂₁ to Q₂₉, and Q₃₁ to Q₃₉ may each independently be selected from hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryl group substituted with at least one selected from a C₁-C₆₀ alkyl group, and a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, and a monovalent non-aromatic condensed heteropolycyclic group.

According to another aspect of an embodiment, an organic light-emitting device includes:
a first electrode,
a second electrode, and
an organic layer located between the first electrode and the second electrode,
wherein the organic layer includes an emission layer and at least one organometallic compound described above.

The organometallic compound in the organic layer may act as a dopant.

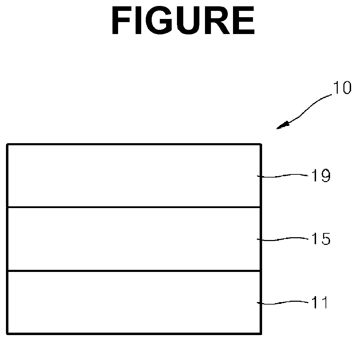

### BRIEF DESCRIPTION OF THE DRAWING

These and/or other aspects will become apparent and more readily appreciated from the following description of the embodiments, taken in conjunction with the FIGURE which is a schematic view of an organic light-emitting device according to an embodiment.

### DETAILED DESCRIPTION OF THE EMBODIMENTS

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

It will be understood that when an element is referred to as being "on" another element, it can be directly in contact with the other element or intervening elements may be present therebetween. In contrast, when an element is referred to as being "directly on" another element, there are no intervening elements present.

It will be understood that, although the terms first, second, third etc. may be used herein to describe various elements, components, regions, layers, and/or sections, these elements, components, regions, layers, and/or sections should not be limited by these terms. These terms are only used to distinguish one element, component, region, layer, or section from another element, component, region, layer, or section. Thus, a first element, component, region, layer, or section discussed below could be termed a second element, component, region, layer, or section without departing from the teachings of the present embodiments.

The terminology used herein is for the purpose of describing particular embodiments only and is not intended to be limiting. As used herein, the singular forms "a," "an," and "the" are intended to include the plural forms as well, unless the context clearly indicates otherwise.

The term "or" means "and/or." It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Unless otherwise defined, all terms (including technical and scientific terms) used herein have the same meaning as commonly understood by one of ordinary skill in the art to which this general inventive concept belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

Exemplary embodiments are described herein with reference to cross section illustrations that are schematic illustrations of idealized embodiments. As such, variations from the shapes of the illustrations as a result, for example, of manufacturing techniques and/or tolerances, are to be expected. Thus, embodiments described herein should not be construed as limited to the particular shapes of regions as illustrated herein but are to include deviations in shapes that result, for example, from manufacturing. For example, a region illustrated or described as flat may, typically, have rough and/or nonlinear features. Moreover, sharp angles that are illustrated may be rounded. Thus, the regions illustrated in the figures are schematic in nature and their shapes are not intended to illustrate the precise shape of a region and are not intended to limit the scope of the present claims.

"About" or "approximately" as used herein is inclusive of the stated value and means within an acceptable range of deviation for the particular value as determined by one of ordinary skill in the art, considering the measurement in question and the error associated with measurement of the particular quantity (*i.e.*, the limitations of the measurement system). For example, "about" can mean within one or more standard deviations, or within ± 30%, 20%, 10%, 5% of the stated value.

An aspect of the present disclosure provides an organometallic compound represented by Formula 1:

Formula 1 M(L₁)ₙ₁(L₂)ₙ₂.

In Formula 1,
M may be a transition metal.

For example, M may be selected from a first-row transition metal of the Periodic Table of Elements, a second-row transition metal of the Periodic Table of Elements, and a third-row transition metal of the Periodic Table of Elements.

In an embodiment, M may be iridium (Ir), platinum (Pt), osmium (Os), titanium (Ti), zirconium (Zr), hafnium (Hf), europium (Eu), terbium (Tb), thulium (Tm), or rhodium (Rh).

In an embodiment, M may be Ir, Pt, Os, or Rh, but embodiments of the present disclosure are not limited thereto.

In Formula 1, L₁ may be a ligand represented by Formula 2 or Formula 3: 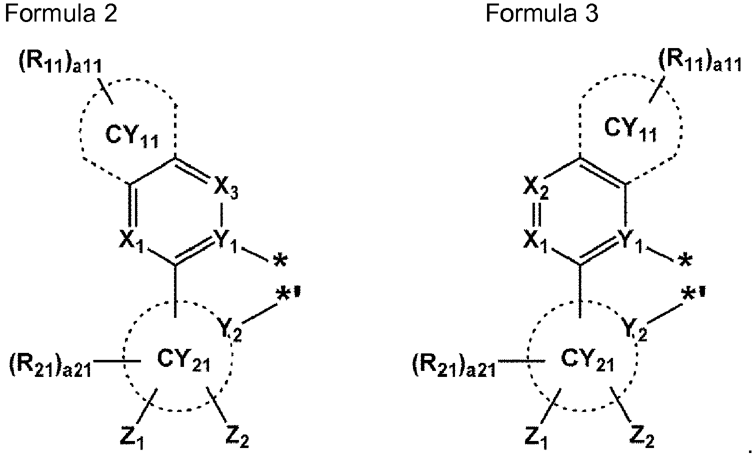 Formulae 2 and 3 are the same as described herein.

In Formula 1, n1 indicates the number of L₁, and may be 1, 2, or 3. When n1 is two or more, two or more groups L₁ may be identical to or different from each other.

In Formula 1, L₂ may be selected from a monodentate ligand, a bidentate ligand, a tridentate ligand, and a tetradentate ligand. L₂ is the same as described herein.

In Formula 1, n2 indicates the number of groups L₂, and may be 0, 1, 2, 3, or 4. When n2 is two or more, two or more groups L₂ may be identical to or different from each other.

In Formula 1, L₁ and L₂ may be different from each other. Thus, when n2 in Formula 1 is not 0, the organometallic compound represented by Formula 1 may be a heteroleptic complex.

In an embodiment, in Formula 1, i) M may be Ir or Os, and n1 + n2 may be 3 or 4; or ii) M may be Pt, and n1 + n2 may be 2.

In one or more embodiments, n2 in Formula 1 may be 1 or 2.

In Formulae 2 and 3, Y₁ and Y₂ may each independently be C or N.

For example, Y₁ may be N, and Y₂ may be C, but embodiments of the present disclosure are not limited thereto.

A bond between Y₁ in each of Formulae 2 and 3 and M in Formula 1 may be a coordinate covalent bond, and a bond between Y₂ in each of Formulae 2 and 3 and M in Formula 1 may be a covalent bond. Therefore, the organometallic compound represented by Formula 1 may be electrically neutral.

In Formulae 2 and 3, CY₁₁ and CY₂₁ may each independently be selected from a C₅-C₃₀ carbocyclic group and a C₁-C₃₀ heterocyclic group.

In an embodiment, ring CY₁₁ and ring CY₂₁ may each independently be selected from a benzene group, a naphthalene group, an anthracene group, a phenanthrene group, a triphenylene group, a pyrene group, a chrysene group, a cyclopentadiene group, a 1,2,3,4-tetrahydronaphthalene group, a thiophene group, a furan group, a selenophene group, an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isooxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, and a 5,6,7,8-tetrahydroquinoline group.

In an embodiment, ring CY₁₁ and ring CY₂₁ may each independently be selected from a benzene group, a pyridine group, a pyrimidine group, a pyridazine group, a furan group, a thiophene group, and a selenophene group, but embodiments of the present disclosure are not limited thereto.

For example, ring CY₁₁ may be a benzene group or a pyridine group. For example, ring CY₂₁ may be a benzene group.

In Formulae 2 and 3, X₁ may be N or C(R₁), X₂ may be N or C(R₂), and X₃ may be N or C(R₃).

For example, X₁ may be N, X₂ may be C(R₂), and X₃ may be C(R₃); X₁ may be N, X₂ may be N, and X₃ may be C(R₃); X₁ may be N, X₂ may be C(R₂), and X₃ may be N; X₁ may be N, X₂ may be N, and X₃ may be N; X₁ may be C(R₁), X₂ may be N, and X₃ may be C(R₃); X₁ may be C(R₁), X₂ may be N, and X₃ may be N; X₁ may be C(R₁), X₂ may be C(R₂), and X₃ may be N; or X₁ may be C(R₁), X₂ may be C(R₂), and X₃ may be C(R₃).

In an embodiment, ring CY₁₁ may be a benzene group, a pyridine group, a furan group, or a selenophene group, X₁ may be C(R₁), X₂ may be C(R₂), and X₃ may be C(R₃).

In Formulae 2 and 3, R₁ to R₃, R₁₁, and R₂₁ may each independently be selected from hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₇-C₆₀ arylalkyl group, a substituted or unsubstituted C₁-C₆₀ heteroaryl group, a substituted or unsubstituted C₁-C₆₀ heteroaryloxy group, a substituted or unsubstituted C₁-C₆₀ heteroarylthio group, a substituted or unsubstituted C₂-C₆₀ heteroarylalkyl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), -Ge(Q₃)(Q₄)(Q₅), -B(Q₆)(Q₇), and -P(=O)(Q₈)(Q₉).

In an embodiment, R₁ to R₃ may each independently be selected from:
hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a methyl group, an ethyl group, an n-propyl group, an iso-propyl group, an n-butyl group, an iso-butyl group, a sec-butyl group, a tert-butyl group, an n-pentyl group, a tert-pentyl group, a neo-pentyl group, an iso-pentyl group, a sec-pentyl group, a 3-pentyl group, and a sec-iso-pentyl group; and
a methyl group, an ethyl group, an n-propyl group, an iso-propyl group, an n-butyl group, an iso-butyl group, a sec-butyl group, a tert-butyl group, an n-pentyl group, a tert-pentyl group, a neo-pentyl group, an iso-pentyl group, a sec-pentyl group, a 3-pentyl group, a sec-iso-pentyl group, each substituted with at least one selected from at least one deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof.

For example, R₁ to R₃ may each independently be selected from:
hydrogen, deuterium, a methyl group, an ethyl group, an n-propyl group, an iso-propyl group, an n-butyl group, an iso-butyl group, a sec-butyl group, and a tert-butyl group; and
a methyl group, an ethyl group, an n-propyl group, an iso-propyl group, an n-butyl group, an iso-butyl group, a sec-butyl group, a tert-butyl group, each substituted with at least one deuterium.

In an embodiment, R₁₁ may be selected from each independently hydrogen, a substituted or unsubstituted C₁-C₆₀ alkyl group, a substituted or unsubstituted C₃-C₁₀ cycloalkyl group, -Si(Q₃)(Q₄)(Q₅), and -Ge(Q₃)(Q₄)(Q₅).

For example, R₁₁ may be selected from:
hydrogen, deuterium, a methyl group, an ethyl group, an n-propyl group, an iso-propyl group, an n-butyl group, an iso-butyl group, a sec-butyl group, a tert-butyl group, an n-pentyl group, a tert-pentyl group, a neo-pentyl group, an iso-pentyl group, a sec-pentyl group, a 3-pentyl group, a sec-iso-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, a tert-decyl group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, -Si(Q₃)(Q₄)(Q₅), and-Ge(Q₃)(Q₄)(Q₅);
a methyl group, an ethyl group, an n-propyl group, an iso-propyl group, an n-butyl group, an iso-butyl group, a sec-butyl group, a tert-butyl group, an n-pentyl group, a tert-pentyl group, a neo-pentyl group, an iso-pentyl group, a sec-pentyl group, a 3-pentyl group, a sec-iso-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, and a tert-decyl group, each substituted with at least one deuterium; and
a cyclopentyl group, a cyclohexyl group, a cycloheptyl group, a cycloctyl group, an adamantanyl group, a norbornanyl group, a norbornenyl group, a cyclopentenyl group, a cyclohexenyl group, and a cycloheptenyl group, each substituted with at least one selected from deuterium and a C₁-C₁₀ alkyl group,
but embodiments of the present disclosure are not limited thereto.

For example, R₁₁ may be selected from:
hydrogen, deuterium, a methyl group, an ethyl group, an n-propyl group, an iso-propyl group, an n-butyl group, an iso-butyl group, a sec-butyl group, a tert-butyl group, an n-pentyl group, a tert-pentyl group, a neo-pentyl group, an iso-pentyl group, a sec-pentyl group, a 3-pentyl group, a sec-iso-pentyl group, a cyclopentyl group, and a cyclohexyl group; and
a methyl group, an ethyl group, an n-propyl group, an iso-propyl group, an n-butyl group, an iso-butyl group, a sec-butyl group, a tert-butyl group, an n-pentyl group, a tert-pentyl group, a neo-pentyl group, an iso-pentyl group, a sec-pentyl group, a 3-pentyl group, and a sec-iso-pentyl group, each substituted with at least one deuterium; and
a cyclopentyl group and a cyclohexyl group, each substituted with at least one selected from deuterium and a C₁-C₅ alkyl group.

In an embodiment, R₂₁ may be selected from:
hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a methyl group, an ethyl group, an n-propyl group, an iso-propyl group, an n-butyl group, an iso-butyl group, a sec-butyl group, a tert-butyl group, an n-pentyl group, a tert-pentyl group, a neo-pentyl group, an iso-pentyl group, a sec-pentyl group, a 3-pentyl group, and a sec-iso-pentyl group; and
a methyl group, an ethyl group, an n-propyl group, an iso-propyl group, an n-butyl group, an iso-butyl group, a sec-butyl group, a tert-butyl group, an n-pentyl group, a tert-pentyl group, a neo-pentyl group, an iso-pentyl group, a sec-pentyl group, a 3-pentyl group, and a sec-iso-pentyl group, each substituted with at least one selected from at least one deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof.

For example, R₂₁ may be selected from:
hydrogen, deuterium, a methyl group, an ethyl group, an n-propyl group, an iso-propyl group, an n-butyl group, an iso-butyl group, a sec-butyl group, and a tert-butyl group; and
a methyl group, an ethyl group, an n-propyl group, an iso-propyl group, an n-butyl group, an iso-butyl group, a sec-butyl group, and a tert-butyl group, each substituted with at least one deuterium.

For example, R₂₁ may be hydrogen.

In Formulae 2 and 3, a11 indicates the number of R₁₁, and may be an integer from 0 to 10. When a11 is two or more, two or more groups R₁₁ may be identical to or different from each other. For example, a11 may be an integer from 0 to 6. For example, a11 may be an integer from 0 to 4.

In Formulae 2 and 3, a21 indicates the number of R₂₁, and may be an integer from 0 to 8. When a21 is two or more, two or more groups R₂₁ may be identical to or different from each other. For example, a21 may be an integer from 0 to 4. For example, a21 may be an integer from 0 to 2.

In Formulae 2 and 3, Z₁ and Z₂ may each independently be selected from a C₁-C₆₀ alkyl group and a deuterium-containing C₁-C₆₀ alkyl group.

In an embodiment, Z₁ and Z₂ may each independently be selected from:
a methyl group, an ethyl group, an n-propyl group, an iso-propyl group, an n-butyl group, an iso-butyl group, a sec-butyl group, a tert-butyl group, an n-pentyl group, a tert-pentyl group, a neo-pentyl group, an iso-pentyl group, a sec-pentyl group, a 3-pentyl group, a sec-iso-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, and a tert-decyl group; and
a methyl group, an ethyl group, an n-propyl group, an iso-propyl group, an n-butyl group, an iso-butyl group, a sec-butyl group, a tert-butyl group, an n-pentyl group, a tert-pentyl group, a neo-pentyl group, an iso-pentyl group, a sec-pentyl group, a 3-pentyl group, a sec-iso-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, and a tert-decyl group, each substituted with at least one deuterium.

For example, Z₁ may be selected from:
an iso-propyl group, an iso-butyl group, a sec-butyl group, a tert-butyl group, a tert-pentyl group, a neo-pentyl group, an iso-pentyl group, a sec-pentyl group, a 3-pentyl group, a sec-iso-pentyl group, an iso-hexyl group, a sec-hexyl group, a tert-hexyl group, an iso-heptyl group, a sec-heptyl group, a tert-heptyl group, an iso-octyl group, a sec-octyl group, a tert-octyl group, an iso-nonyl group, a sec-nonyl group, a tert-nonyl group, an iso-decyl group, a sec-decyl group, and a tert-decyl group; and
an iso-propyl group, an iso-butyl group, a sec-butyl group, a tert-butyl group, a tert-pentyl group, a neo-pentyl group, an iso-pentyl group, a sec-pentyl group, a 3-pentyl group, a sec-iso-pentyl group, an iso-hexyl group, a sec-hexyl group, a tert-hexyl group, an iso-heptyl group, a sec-heptyl group, a tert-heptyl group, an iso-octyl group, a sec-octyl group, a tert-octyl group, an iso-nonyl group, a sec-nonyl group, a tert-nonyl group, an iso-decyl group, a sec-decyl group, and a tert-decyl group, each substituted with at least one deuterium, and
Z₂ may be selected from a methyl group, an ethyl group, an n-propyl group, an iso-propyl group, an n-butyl group, an iso-butyl group, a sec-butyl group, and a tert-butyl group.

In Formulae 2 and 3, Z₁ and Z₂ may be different from each other.

In an embodiment, the number of carbon atoms included in Z₁ may be greater than that of carbon atoms included in Z₂.

In an embodiment, R₂₁ may be hydrogen, and

Z₁ and Z₂ may each independently be selected from:
a methyl group, an ethyl group, an n-propyl group, an iso-propyl group, an n-butyl group, an iso-butyl group, a sec-butyl group, a tert-butyl group, an n-pentyl group, a tert-pentyl group, a neo-pentyl group, an iso-pentyl group, a sec-pentyl group, a 3-pentyl group, a sec-iso-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, and a tert-decyl group; and
a methyl group, an ethyl group, an n-propyl group, an iso-propyl group, an n-butyl group, an iso-butyl group, a sec-butyl group, a tert-butyl group, an n-pentyl group, a tert-pentyl group, a neo-pentyl group, an iso-pentyl group, a sec-pentyl group, a 3-pentyl group, a sec-iso-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, and a tert-decyl group, each substituted with at least one deuterium.

In an embodiment, a group represented by 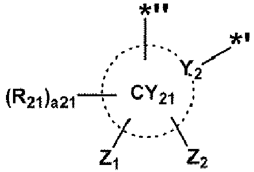 in Formulae 2 and 3 may be selected from groups represented by Formulae CY1-1 to CY1-6: 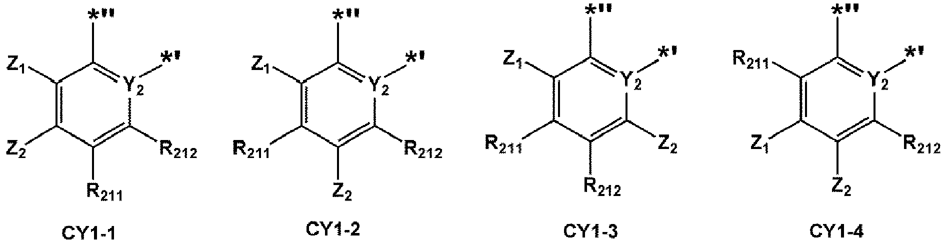 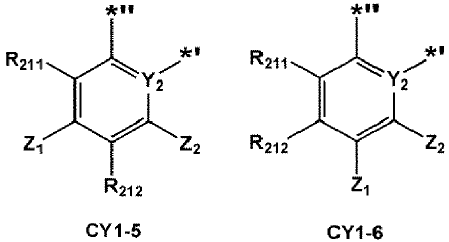 In Formulae CY1-1 to CY1-6,
R₂₁₁ and R₂₁₂ are each independently defined the same as R₂₁,
Z₁, Z₂, and Y₂ are each independently the same as described herein,
*' indicates a binding site to M in Formula 1, and
*" indicates a binding site to a neighboring group in Formula 2.

For example, in Formulae CY1-1 to CY1-6, the number of carbon atoms included in Z₁ may be greater than that of carbon atoms included in Z₂.

For example, in Formulae CY1-1 to CY1-6,
Z₁ may be selected from:
an ethyl group, an iso-propyl group, an iso-butyl group, a sec-butyl group, a tert-butyl group, a tert-pentyl group, a neo-pentyl group, an iso-pentyl group, a sec-pentyl group, a 3-pentyl group, and a sec-iso-pentyl group; and
an ethyl group, an iso-propyl group, an iso-butyl group, a sec-butyl group, a tert-butyl group, a tert-pentyl group, a neo-pentyl group, an iso-pentyl group, a sec-pentyl group, a 3-pentyl group, and a sec-iso-pentyl group, each substituted with at least one deuterium, and
Z₂ may be a methyl group.

In Formula 1, L₂ may be a bidentate ligand linked to M in Formula 1 via O, S, N, C, P, Si, or As.

In an embodiment, in Formula 1, L₂ may be a bidentate ligand represented by Formula 4: 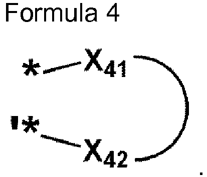 In Formula 4,
X₄₁ and X₄₂ may each independently be O, S, N, C, P, Si, or As,
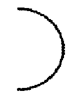 indicates an atomic group linking X₄₁ and X₄₂ to each other, and
* and *' each indicate a binding site to M in Formula 1.

For example, in Formula 4, X₄₁ and X₄₂ may each independently be O, but embodiments of the present disclosure are not limited thereto.

In one or more embodiments, in Formula 1, L₂ may be a monodentate ligand, such as I⁻, Br⁻, Cl⁻, sulfide, nitrate, azide, hydroxide, cyanate, isocyanate, thiocyanate, water, acetonitrile, pyridine, ammonia, carbon monoxide, P(Ph)₃, P(Ph)₂CH₃, PPh(CH₃)₂, and P(CH₃)₃, but embodiments of the present disclosure are not limited thereto.

In one or more embodiments, in Formula 1, L₂ may be a bidentate ligand, such as oxalate, acetylacetonate, picolinic acid, 1,2-bis(diphenylphosphino)ethane, 1,1-bis(diphenylphosphino)methane, glycinate, and ethylenediamine, but embodiments of the present disclosure are not limited thereto.

In one or more embodiments, in Formula 1, L₂ may be selected from groups represented by Formulae 4A to 4F: 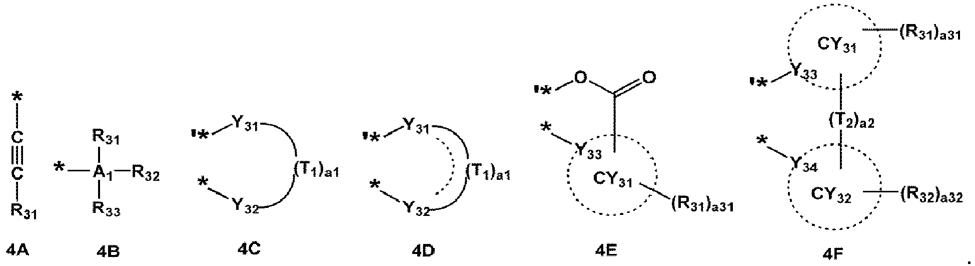 In Formulae 4A to 4F,
Y₃₁ may be selected from O, N, N(R₃₄), P(R₃₄)(R₃₅), and As(R₃₄)(R₃₅),
Y₃₂ may be selected from O, N, N(R₃₆), P(R₃₆)(R₃₇), and As(R₃₆)(R₃₇),
T₁ may be selected from a single bond, a double bond, *-C(R₃₄)(R₃₅)-*', *-C(R₃₄)=C(R₃₅)-*', *=C(R₃₄)-*', *-C(R₃₄)=*', *=C(R₃₄)-C(R₃₅)=C(R₃₆)-*', *-C(R₃₄)=C(R₃₅)-C(R₃₆)=*', and *-N(R₃₄)-*',
Y₃₃ and Y₃₄ may each independently be C or N,
T₂ may be a single bond, a double bond, O, S, C(R₃₄)(R₃₅), Si(R₃₄)(R₃₅), or N(R₃₄),
ring CY₃₁ and ring CY₃₂ may each independently be selected from a C₅-C₃₀ carbocyclic group and a C₁-C₃₀ heterocyclic group,
A₁ may be P or As,
R₃₁ to R₃₇ are each independently defined the same as R₁,
a1 and a2 may each independently be an integer from 0 to 10, and
* and *' each indicate a binding site to M in Formula 1.

For example, in Formula 1, L₂ may be a group represented by Formula 4D,
Y₃₁ and Y₃₂ may each independently be O,
T₁ may be selected from *=C(R₃₄)-C(R₃₅)=C(R₃₆)-*' and *-C(R₃₄)=C(R₃₅)-C(R₃₆)=*',
a1 may be 1,
R₃₄ to R₃₆ may each independently be selected from:
   a methyl group, an ethyl group, an n-propyl group, an iso-propyl group, an n-butyl group, an iso-butyl group, a sec-butyl group, a tert-butyl group, an n-pentyl group, a tert-pentyl group, a neo-pentyl group, an iso-pentyl group, a sec-pentyl group, a 3-pentyl group, a sec-iso-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, and a tert-decyl group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a phenyl group, a biphenyl group, a terphenyl group, a naphthyl group, a pyridinyl group, a pyrimidinyl group, a carbazolyl group, a fluorenyl group, a dibenzosilolyl group, a dibenzofuranyl group, a dibenzothiophenyl group, -Si(Q₃)(Q₄)(Q₅), and -Ge(Q₃)(Q₄)(Q₅);
   a methyl group, an ethyl group, an n-propyl group, an iso-propyl group, an n-butyl group, an iso-butyl group, a sec-butyl group, a tert-butyl group, an n-pentyl group, a tert-pentyl group, a neo-pentyl group, an iso-pentyl group, a sec-pentyl group, a 3-pentyl group, a sec-iso-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, and a tert-decyl group, each substituted with at least one deuterium; and
   a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a phenyl group, a biphenyl group, a terphenyl group, a naphthyl group, a pyridinyl group, a pyrimidinyl group, a carbazolyl group, a fluorenyl group, a dibenzosilolyl group, a dibenzofuranyl group, and a dibenzothiophenyl group, each substituted with at least one selected from deuterium, a C₁-C₁₀ alkyl group, a phenyl group, and a biphenyl group.

In an embodiment, in Formula 1, L₂ may be a group represented by Formula 4D-1, but embodiments of the present disclosure are not limited thereto: 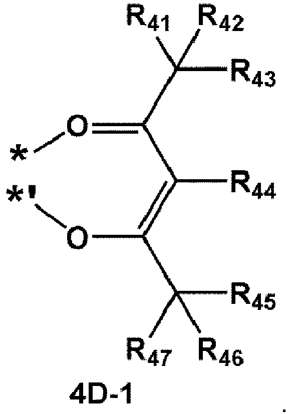 In Formula 4D-1,
R₄₄ is defined the same as R₁,
R₄₁ to R₄₃ and R₄₅ to R₄₇ may each independently be selected from hydrogen, deuterium, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₇-C₆₀ arylalkyl group, a substituted or unsubstituted C₁-C₆₀ heteroaryl group, a substituted or unsubstituted C₁-C₆₀ heteroaryloxy group, a substituted or unsubstituted C₁-C₆₀ heteroarylthio group, a substituted or unsubstituted C₂-C₆₀ heteroarylalkyl group, a substituted or unsubstituted monovalent non-aromatic condensed polycyclic group, and a substituted or unsubstituted monovalent non-aromatic condensed heteropolycyclic group,
at least one substituent of the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₇-C₆₀ arylalkyl group, the substituted C₁-C₆₀ heteroaryl group, the substituted C₁-C₆₀ heteroaryloxy group, the substituted C₁-C₆₀ heteroarylthio group, the substituted C₂-C₆₀ heteroarylalkyl group, the substituted monovalent non-aromatic condensed polycyclic group, and the substituted monovalent non-aromatic condensed heteropolycyclic group may be selected from:
   deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
   a C₁-C₆₀ alkyl group, a C₂-C₆₀ alkenyl group, a C₂-C₆₀ alkynyl group, and a C₁-C₆₀ alkoxy group, each substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, a monovalent non-aromatic condensed heteropolycyclic group, -N(Q₁₁)(Q₁₂),-Si(Q₁₃)(Q₁₄)(Q₁₅), -Ge(Q₁₃)(Q₁₄)(Q₁₅), -B(Q₁₆)(Q₁₇), -P(=O)(Q₁₈)(Q₁₉), and-P(Q₁₈)(Q₁₉);
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, and a monovalent non-aromatic condensed heteropolycyclic group;
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, and a monovalent non-aromatic condensed heteropolycyclic group, each substituted with at least one selected from deuterium, -F, -Cl, -Br, -I, -CD₃,-CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, a monovalent non-aromatic condensed heteropolycyclic group, -N(Q₂₁)(Q₂₂),-Si(Q₂₃)(Q₂₄)(Q₂₅), -Ge(Q₂₃)(Q₂₄)(Q₂₅), -B(Q₂₆)(Q₂₇), -P(=O)(Q₂₈)(Q₂₉), and-P(Q₂₈)(Q₂₉); and
   -N(Q₃₁)(Q₃₂), -Si(Q₃₃)(Q₃₄)(Q₃₅), -Ge(Q₃₃)(Q₃₄)(Q₃₅), -B(Q₃₆)(Q₃₇),-P(=O)(Q₃₈)(Q₃₉), and -P(Q₃₈)(Q₃₉), and
   Q₁₁ to Q₁₉, Q₂₁ to Q₂₉, and Q₃₁ to Q₃₉ may each independently be selected from hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryl group substituted with at least one selected from a C₁-C₆₀ alkyl group, and a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, and a monovalent non-aromatic condensed heteropolycyclic group.

For example, in Formula 4D-1, R₄₁ to R₄₇ may each independently be selected from:
hydrogen and deuterium;
a C₁-C₂₀ alkyl group and a C₁-C₂₀ alkoxy group, each unsubstituted or substituted with at least one selected from deuterium, -F, -Cl, -Br, -I, -CD₃,-CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a phenyl group, a biphenyl group, a naphthyl group, a pyridinyl group, and a pyrimidinyl group; and
a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a phenyl group, a biphenyl group, a C₁-C₂₀ alkylphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group, each unsubstituted or substituted with at least one selected from deuterium, -F, -Cl, -Br, -I, -CD₃, -CD₂H, -CDH₂, -CF₃, -CF₂H,-CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a phenyl group, a biphenyl group, a C₁-C₂₀ alkylphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group.

In an embodiment, in Formula 4D-1, R₄₁ to R₄₇ may each independently be selected from:
hydrogen and deuterium;
a methyl group, an ethyl group, an n-propyl group, an iso-propyl group, an n-butyl group, an iso-butyl group, a sec-butyl group, a tert-butyl group, an n-pentyl group, an iso-pentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, a tert-decyl group, a methoxy group, an ethoxy group, a propoxy group, a butoxy group, a pentoxy group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a phenyl group, a biphenyl group, a terphenyl group, a naphthyl group, a pyridinyl group, a pyrimidinyl group, a carbazolyl group, a fluorenyl group, a dibenzosilolyl group, a dibenzofuranyl group, and a dibenzothiophenyl group;
a methyl group, an ethyl group, an n-propyl group, an iso-propyl group, an n-butyl group, an iso-butyl group, a sec-butyl group, a tert-butyl group, an n-pentyl group, an iso-pentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, and a tert-decyl group, each substituted with at least one selected from deuterium, -F, and a cyano group; and
a methoxy group, an ethoxy group, a propoxy group, a butoxy group, a pentoxy group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a phenyl group, a biphenyl group, a terphenyl group, a naphthyl group, a pyridinyl group, a pyrimidinyl group, a carbazolyl group, a fluorenyl group, a dibenzosilolyl group, a dibenzofuranyl group, and a dibenzothiophenyl group, each substituted with at least one selected from deuterium, -F, a cyano group, and a C₁-C₁₀ alkyl group.

For example, in Formula 4D-1, R₄₄ may be hydrogen or deuterium. For example, in Formula 4D-1, R₄₄ may be hydrogen.

The organometallic compound represented by Formula 1 may emit visible light having a maximum emission wavelength of, for example, 450 nanometers (nm) or more and 700 nm or less.

The term "an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, and an azadibenzothiophene 5,5-dioxide group" as used herein each refer to a heterocyclic group having the same backbone as each of "an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluorene-9-one group, and a dibenzothiophene 5,5-dioxide group", respectively, wherein at least one of carbon atoms constituting a ring is substituted with nitrogen.

In an embodiment, the organometallic compound may be selected from Compounds 1 to 200, but embodiments of the present disclosure are not limited thereto:

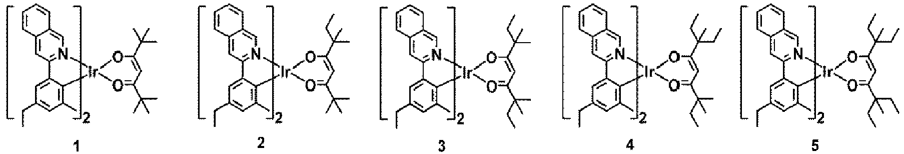

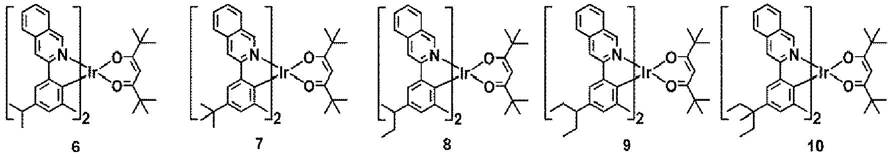

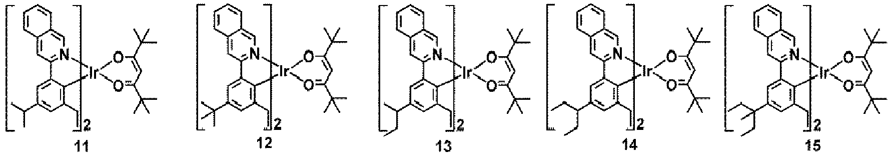

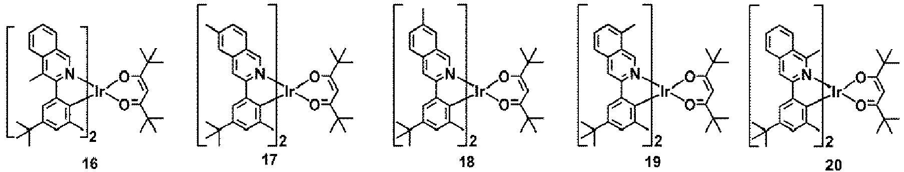

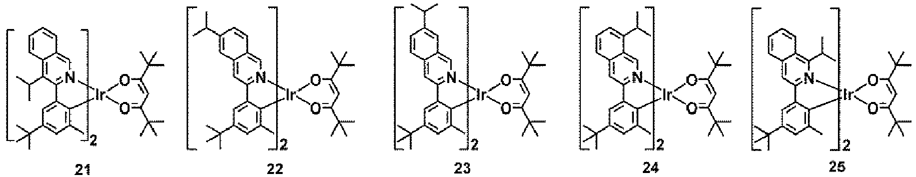

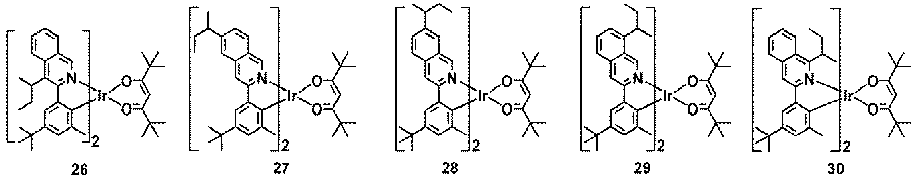

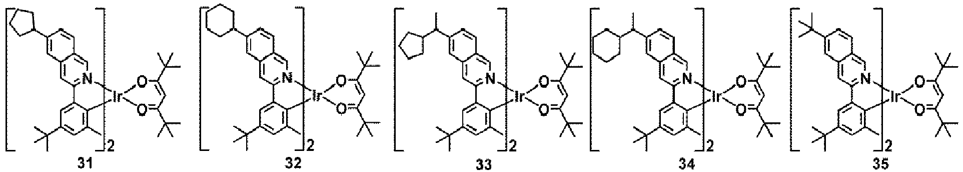

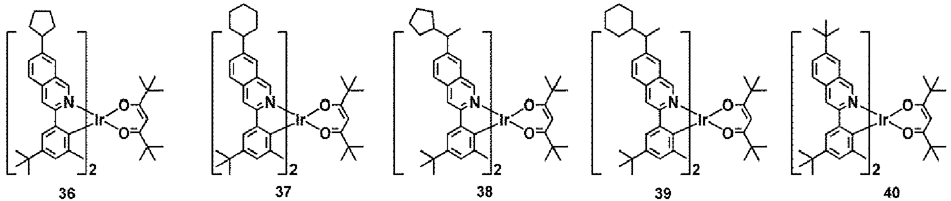

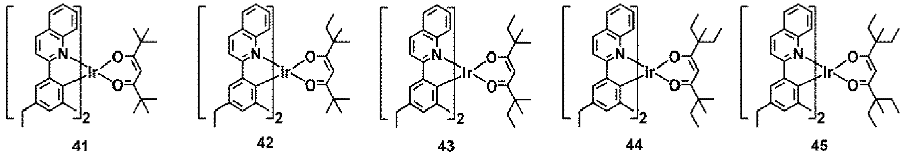

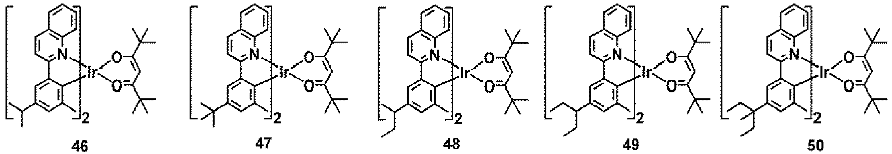

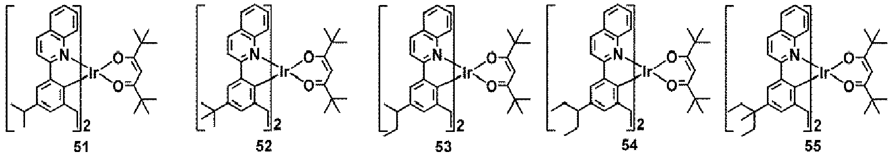

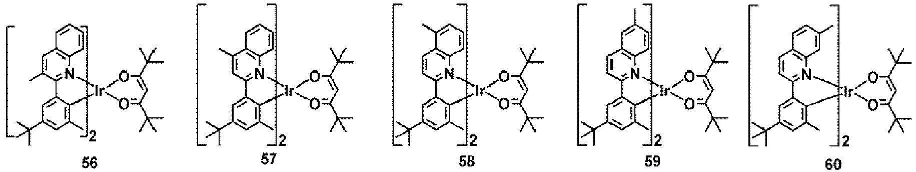

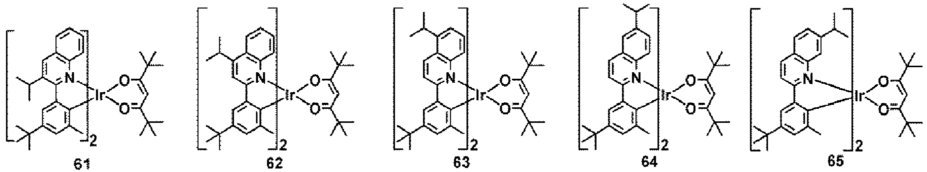

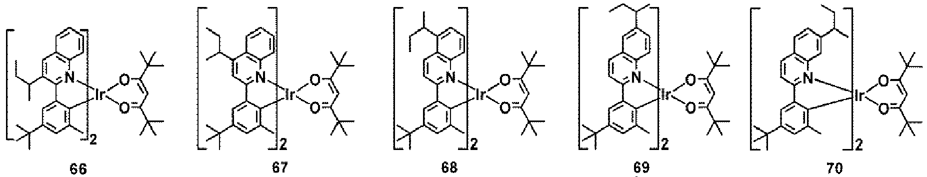

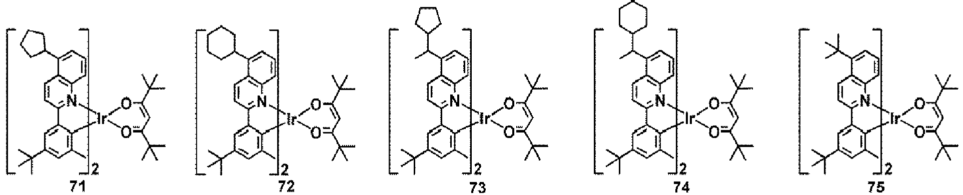

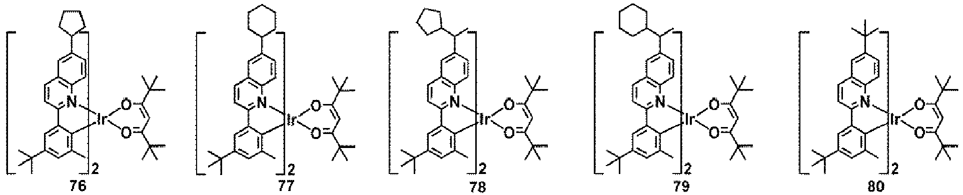

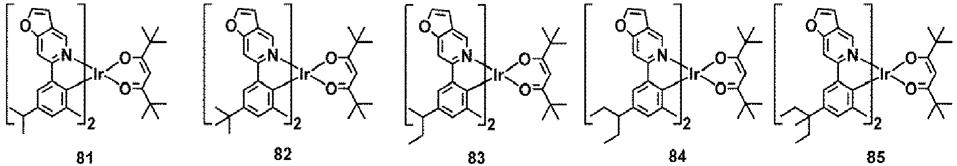

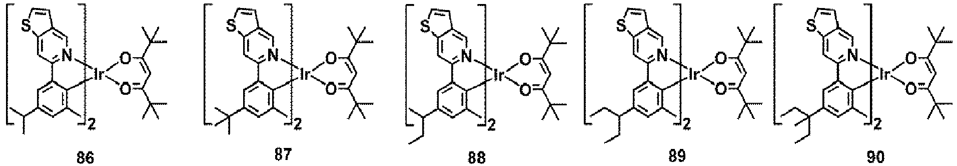

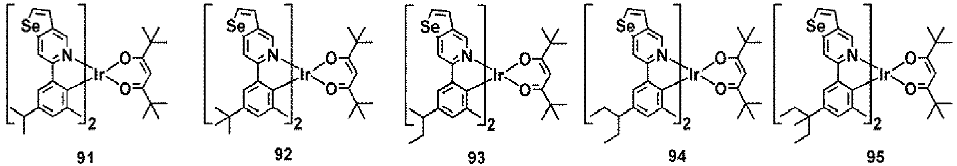

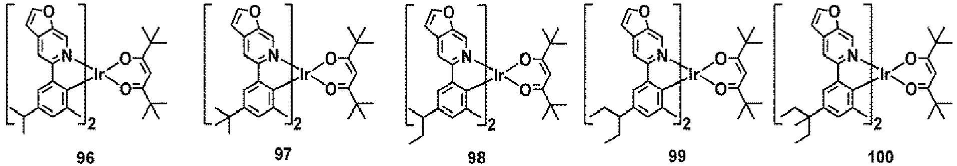

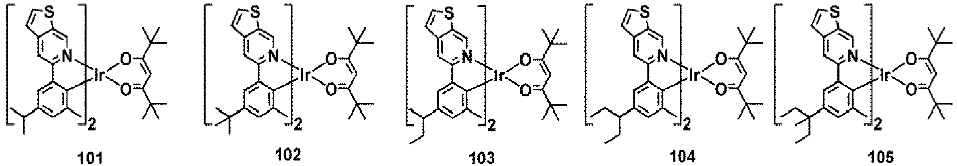

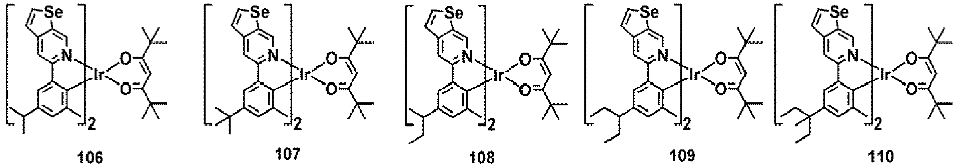

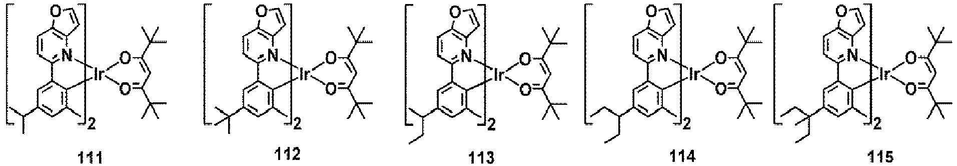

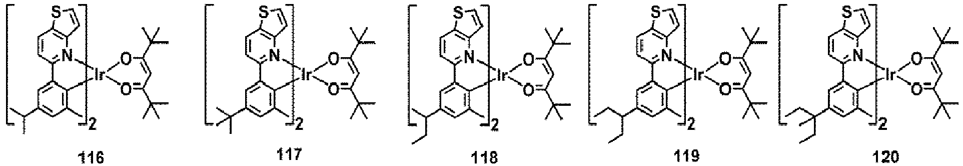

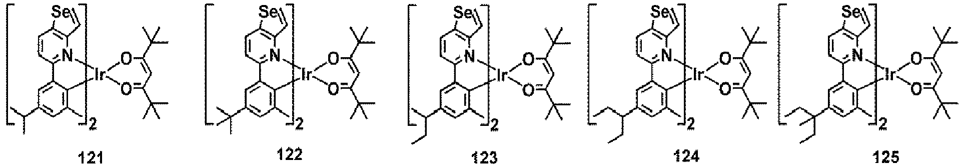

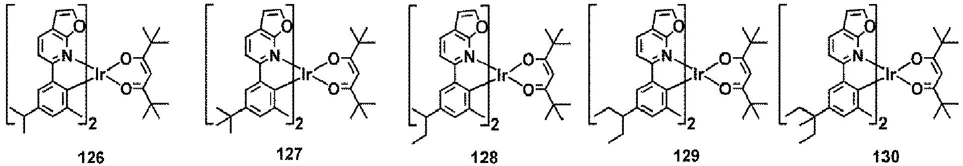

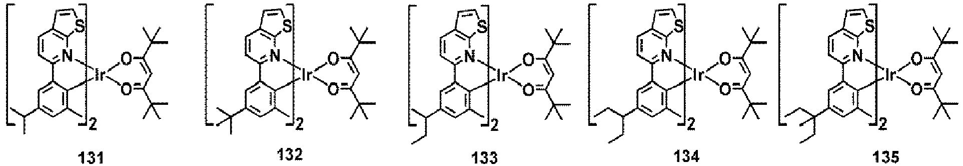

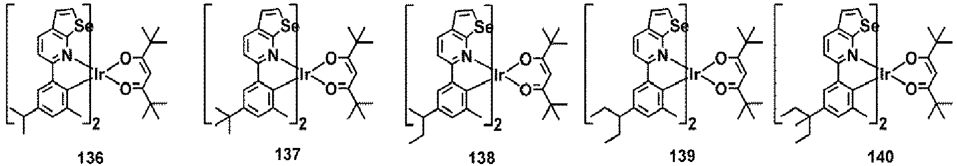

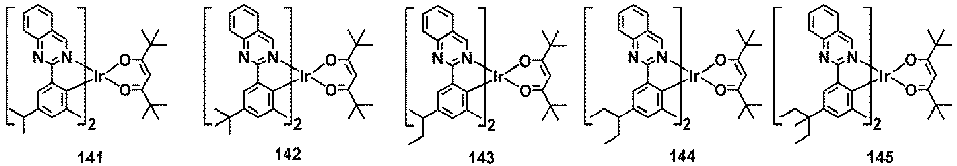

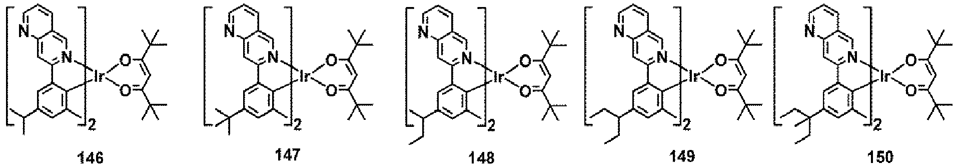

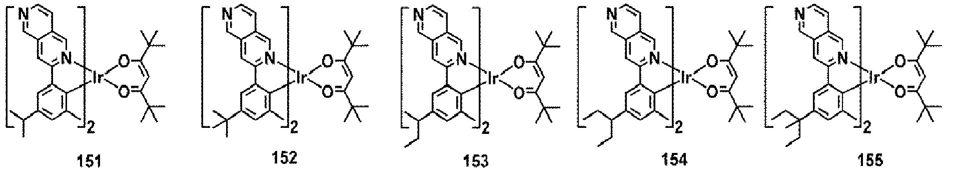

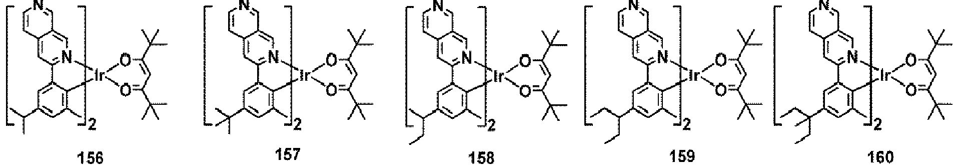

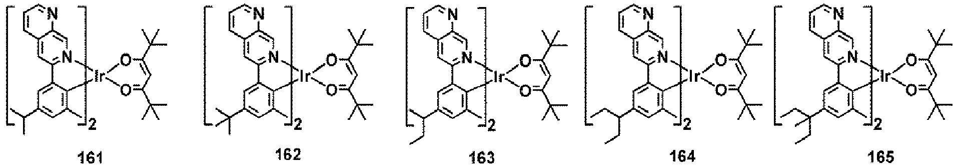

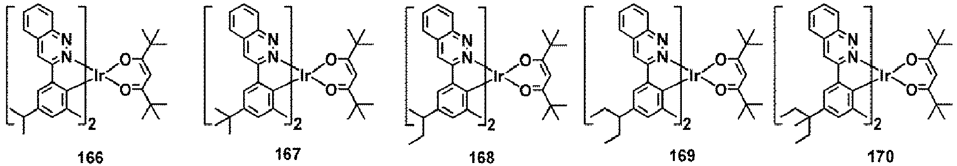

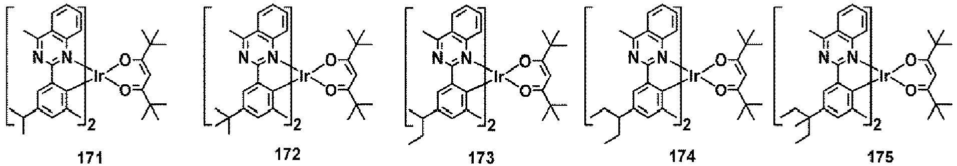

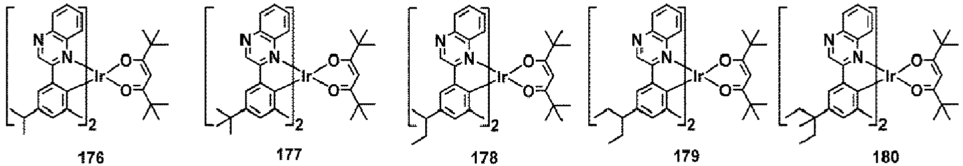

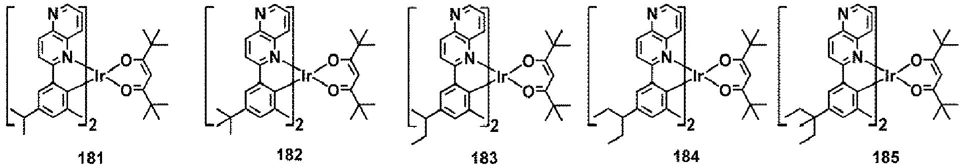

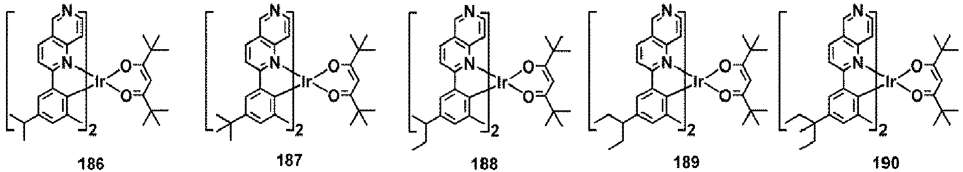

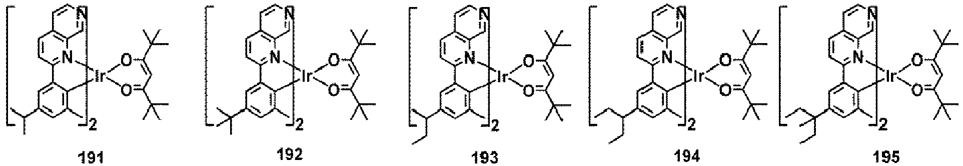

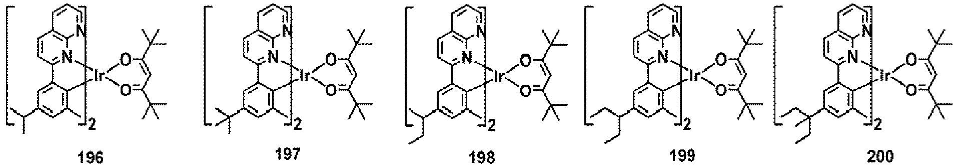

In the organometallic compound represented by Formula 1, L₁ may be a ligand represented by Formula 2 or Formula 3, and n1, which indicates the number of L₁, may be 1, 2, or 3. That is, the organometallic compound includes a ligand linked to M which is a metal, and the organometallic compound includes at least one of ligands represented by Formulae 2 and Formula 3.

In Formula 2, an upper ligand may have a carbocyclic ring or a heterocyclic ring, each condensed with at least two rings. In this regard, the size of a conjugated structure may be increased, resulting in an improved internal quantum luminescence efficiency which is good for the emission of a wavelength band of high efficiency.

In Formula 2, Z₁ and Z₂ in a lower ligand may each independently be selected from a C₁-C₆₀ alkyl group and a deuterium-containing C₁-C₆₀ alkyl group. In this regard, two ligands of a highest occupied molecular orbital (HOMO) condensed ring each affect half-width improvement of the emission wavelength and control of the sublimation temperature of a dopant. Compared to an analog where a cycloalkyl group is substituted, the organometallic compound may have a stable chemical structure with minimized occurrence of side reactions before/after synthesis. Thus, a highly reliable dopant may be prepared, and accordingly, an improved lifespan of a device is also expected from such a stable chemical structure described above.

In addition, in Formula 2, Z₁ and Z₂ may be different from each other. Thus, the size of only the substituent at the outside of the dopant may be optionally controlled, and when an interaction with a host material is increased, an improved emission efficiency and an improved lifespan of a device may be resulted.

Some of the compounds of the organometallic compound represented by Formula 1 are subjected to measurement of a HOMO energy level, a lowest unoccupied molecular orbital (LUMO) level, a band gap, a singlet (S₁) energy level, and a triplet (T₁) energy level by using a Density Functional Theory (DFT) method of a Gaussian 09 program that is structurally optimized at a level of B3LYP, and the results are shown in Table 1.

**Table 1**

| Compound No. | HOMO (eV) | LUMO (eV) | S₁ (eV) | T₁ (eV) |
|---|---|---|---|---|
| 1 | -4.60 | -1.67 | 2.30 | 2.16 |
| 6 | -4.60 | -1.68 | 2.30 | 2.16 |
| 7 | -4.61 | -1.68 | 2.31 | 2.17 |
| 9 | -4.59 | -1.67 | 2.30 | 2.15 |
| 28 | -4.53 | -1.58 | 2.33 | 2.18 |
| 41 | -4.70 | -1.81 | 2.02 | 2.25 |
| 46 | -4.70 | -1.81 | 2.02 | 2.25 |
| 47 | -4.70 | -1.81 | 2.03 | 2.25 |
| 49 | -4.68 | -1.80 | 2.02 | 2.24 |
| 69 | -4.64 | -1.74 | 2.04 | 2.26 |

From Table 1, it is confirmed that the organometallic compound represented by Formula 1 has such electric characteristics that are suitable for use in an electric device, for example, for use as a dopant for an organic light-emitting device.

Synthesis methods of the organometallic compound represented by Formula 1 may be understood by one of ordinary skill in the art by referring to Synthesis Examples provided below.

The organometallic compound represented by Formula 1 is suitable for use in an organic layer of an organic light-emitting device, for example, for use as a dopant in an emission layer of the organic layer. Thus, another aspect of the present disclosure provides an organic light-emitting device that includes: a first electrode; a second electrode; and an organic layer that is disposed between the first electrode and the second electrode, wherein the organic layer includes an emission layer and at least one organometallic compound represented by Formula 1.

The organic light-emitting device may have, due to the inclusion of an organic layer including the organometallic compound represented by Formula 1, a low driving voltage, high efficiency, high power, high quantum efficiency, a long lifespan, a low roll-off ratio, and excellent color purity.

The organometallic compound of Formula 1 may be used between a pair of electrodes of an organic light-emitting device. For example, the organometallic compound represented by Formula 1 may be included in the emission layer. In this regard, the organometallic compound may act as a dopant, and the emission layer may further include a host (that is, an amount of the organometallic compound represented by Formula 1 is smaller than an amount of the host). The emission layer may emit red light, for example, red light having a maximum emission wavelength of 550 nm or more (for example, 550 nm or more and 900 nm or less).

The expression "(an organic layer) includes at least one of organometallic compounds" used herein may include an embodiment in which "(an organic layer) includes identical organometallic compounds represented by Formula 1" and an embodiment in which "(an organic layer) includes two or more different organometallic compounds represented by Formula 1."

For example, the organic layer may include, as the organometallic compound, only Compound 1. In this embodiment, Compound 1 may be included in an emission layer of the organic light-emitting device. In one or more embodiments, the organic layer may include, as the organometallic compound, Compound 1 and Compound 2. In this embodiment, Compound 1 and Compound 2 may be included in an identical layer (for example, Compound 1 and Compound 2 may be included in an emission layer).

The first electrode may be an anode, which is a hole injection electrode, and the second electrode may be a cathode, which is an electron injection electrode; or the first electrode may be a cathode, which is an electron injection electrode, and the second electrode may be an anode, which is a hole injection electrode.

In an embodiment, in the organic light-emitting device, the first electrode may be an anode, and the second electrode may be a cathode, and the organic layer may further include a hole transport region disposed between the first electrode and the emission layer and an electron transport region disposed between the emission layer and the second electrode, wherein the hole transport region may include a hole injection layer, a hole transport layer, an electron blocking layer, a buffer layer, or any combination thereof, and wherein the electron transport region may include a hole blocking layer, an electron transport layer, an electron injection layer, or any combination thereof.

The term "organic layer" as used herein refers to a single layer and/or a plurality of layers disposed between the first electrode and the second electrode of the organic light-emitting device. The "organic layer" may include, in addition to an organic compound, an organometallic complex including metal.

The FIGURE is a schematic view of an organic light-emitting device 10 according to an embodiment. Hereinafter, the structure of an organic light-emitting device according to an embodiment and a method of manufacturing an organic light-emitting device according to an embodiment will be described in connection with the FIGURE. The organic light-emitting device 10 includes a first electrode 11, an organic layer 15, and a second electrode 19, which are sequentially stacked.

A substrate may be additionally disposed under the first electrode 11 or above the second electrode 19. For use as the substrate, any substrate that is used in general organic light-emitting devices may be used, and the substrate may be a glass substrate or a transparent plastic substrate, each having excellent mechanical strength, thermal stability, transparency, surface smoothness, ease of handling, and water resistance.

The first electrode 11 may be formed by depositing or sputtering a material for forming the first electrode 11 on the substrate. The first electrode 11 may be an anode. The material for forming the first electrode 11 may be selected from materials with a high work function to facilitate hole injection. The first electrode 11 may be a reflective electrode, a semi-transmissive electrode, or a transmissive electrode. The material for forming the first electrode may be, for example, indium tin oxide (ITO), indium zinc oxide (IZO), tin oxide (SnO₂), and zinc oxide (ZnO). In one or more embodiments, magnesium (Mg), aluminum (Al), aluminum-lithium (Al-Li), calcium (Ca), magnesium-indium (Mg-In), or magnesium-silver (Mg-Ag) may be used as the material for forming the first electrode.

The first electrode 11 may have a single-layered structure or a multi-layered structure including two or more layers. For example, the first electrode 11 may have a three-layered structure of ITO/Ag/ITO, but the structure of the first electrode 110 is not limited thereto.

The organic layer 15 is disposed on the first electrode 11.

The organic layer 15 may include a hole transport region, an emission layer, and an electron transport region.

The hole transport region may be disposed between the first electrode 11 and the emission layer.

The hole transport region may include a hole injection layer, a hole transport layer, an electron blocking layer, a buffer layer, or any combination thereof.

The hole transport region may include only either a hole injection layer or a hole transport layer. In one or more embodiments, the hole transport region may have a hole injection layer/hole transport layer structure or a hole injection layer/hole transport layer/electron blocking layer structure, which are sequentially stacked in this stated order from the first electrode 11.

A hole injection layer may be formed on the first electrode 11 by using one or more suitable methods selected from vacuum deposition, spin coating, casting, or Langmuir-Blodgett (LB) deposition.

When a hole injection layer is formed by vacuum deposition, the deposition conditions may vary according to a compound that is used to form the hole injection layer, and the structure and thermal characteristics of the hole injection layer. For example, the deposition conditions may include a deposition temperature of about 100 °C to about 500 °C, a vacuum pressure of about 10⁻⁸ torr to about 10⁻³ torr, and a deposition rate of about 0.01 Angstroms per second (Å/sec) to about 100 Å/sec. However, the deposition conditions are not limited thereto.

When the hole injection layer is formed using spin coating, coating conditions may vary according to the material used to form the hole injection layer, and the structure and thermal properties of the hole injection layer. For example, a coating speed may be from about 2,000 revolutions per minute (rpm) to about 5,000 rpm, and a temperature at which a heat treatment is performed to remove a solvent after coating may be from about 80 °C to about 200 °C. However, the coating conditions are not limited thereto.

Conditions for forming a hole transport layer and an electron blocking layer may be understood by referring to conditions for forming the hole injection layer.

The hole transport region may include at least one selected from m-MTDATA, TDATA, 2-TNATA, NPB, β-NPB, TPD, Spiro-TPD, Spiro-NPB, methylated-NPB, TAPC, HMTPD, 4,4',4"-tris(N-carbazolyl)triphenylamine (TCTA), polyaniline/dodecylbenzene sulfonic acid (PANI/DBSA), poly(3,4-ethylenedioxythiophene)/poly(4-styrene sulfonate) (PEDOT/PSS), polyaniline/camphor sulfonic acid (PANI/CSA), polyaniline/poly(4-styrene sulfonate) (PANI/PSS), a compound represented by Formula 201, and a compound represented by Formula 202:

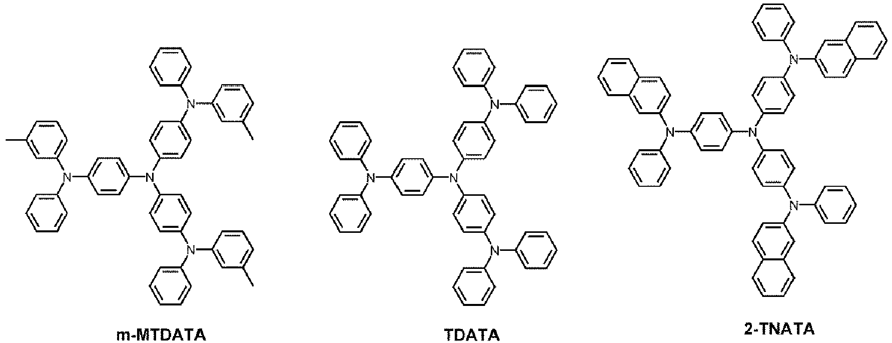

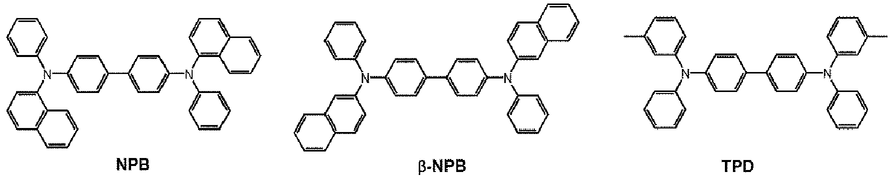

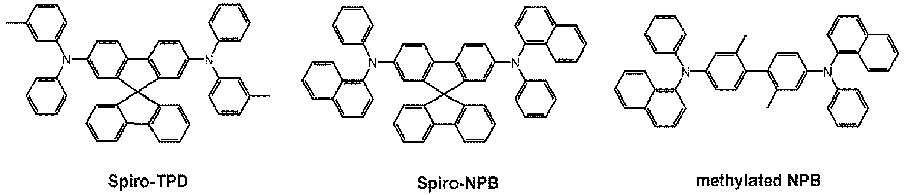

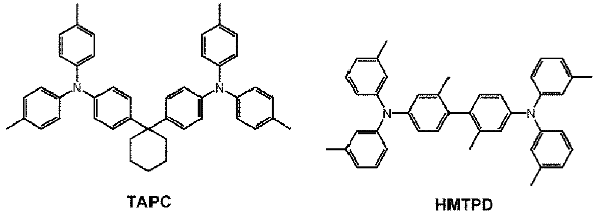

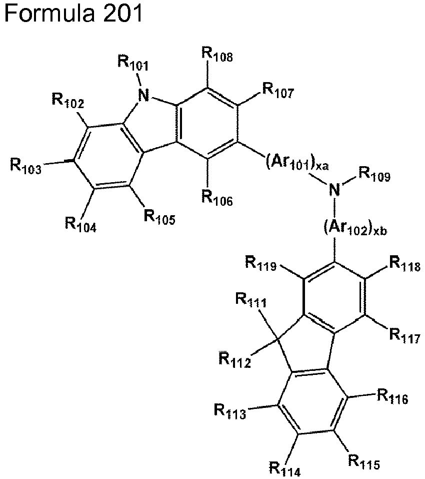

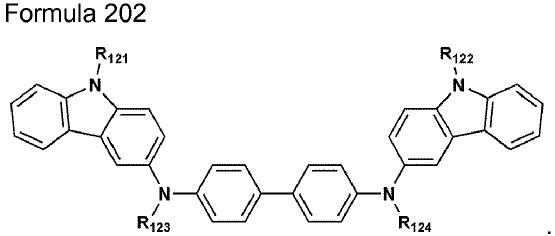

Ar₁₀₁ and Ar₁₀₂ in Formula 201 may each independently be selected from:
a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, and a pentacenylene group; and
a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, and a pentacenylene group, each substituted with at least one selected from deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ cycloalkenyl group, a C₁-C₁₀ heterocycloalkyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, and a monovalent non-aromatic condensed heteropolycyclic group.

In Formula 201, xa and xb may each independently be an integer from 0 to 5, or may be 0, 1, or 2. For example, xa is 1 and xb is 0, but xa and xb are not limited thereto.

R₁₀₁ to R₁₀₈, R₁₁₁ to R₁₁₉, and R₁₂₁ to R₁₂₄ in Formulae 201 and 202 may each independently be selected from:
hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group (for example, a methyl group, an ethyl group, a propyl group, a butyl group, a pentyl group, a hexyl group, and so on), and a C₁-C₁₀ alkoxy group (for example, a methoxy group, an ethoxy group, a propoxy group, a butoxy group, a pentoxy group, and so on);
a C₁-C₁₀ alkyl group or a C₁-C₁₀ alkoxy group, each substituted with at least one selected from deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, and a phosphoric acid group or a salt thereof;
a phenyl group, a naphthyl group, an anthracenyl group, a fluorenyl group, and a pyrenyl group; and
a phenyl group, a naphthyl group, an anthracenyl group, a fluorenyl group, and a pyrenyl group, each substituted with at least one selected from deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, and a C₁-C₁₀ alkoxy group,
but embodiments of the present disclosure are not limited thereto.

In Formula 201, R₁₀₉ may be selected from:
a phenyl group, a naphthyl group, an anthracenyl group, and a pyridinyl group; and
a phenyl group, a naphthyl group, an anthracenyl group, and a pyridinyl group, each substituted with at least one selected from deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, a naphthyl group, an anthracenyl group, and a pyridinyl group.

In an embodiment, the compound represented by Formula 201 may be represented by Formula 201A, but embodiments of the present disclosure are not limited thereto:

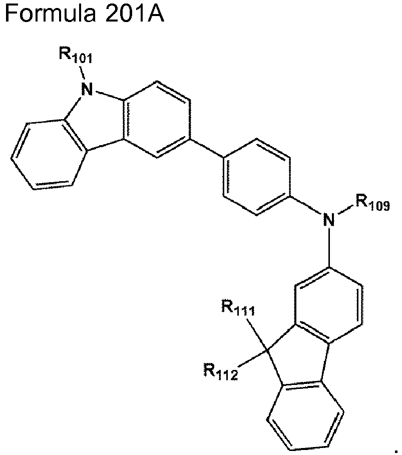

In Formula 201A, R₁₀₁, R₁₁₁, R₁₁₂, and R₁₀₉ are each independently the same as described herein.

For example, the compound represented by Formula 201 and the compound represented by Formula 202 may include Compounds HT1 to HT20, but are not limited thereto:

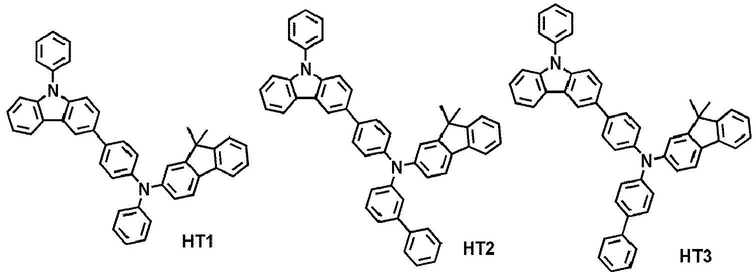

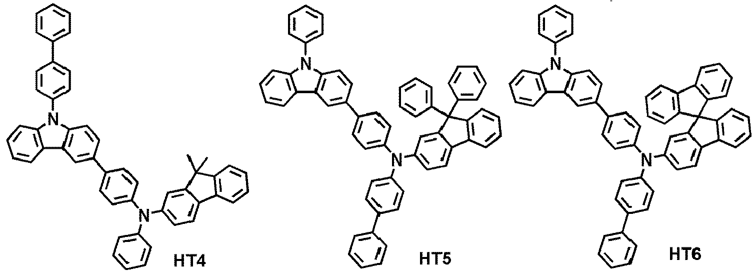

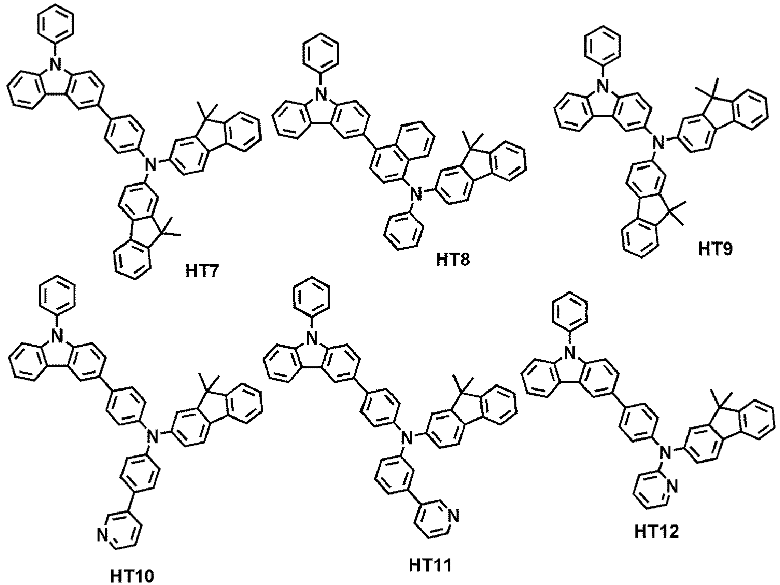

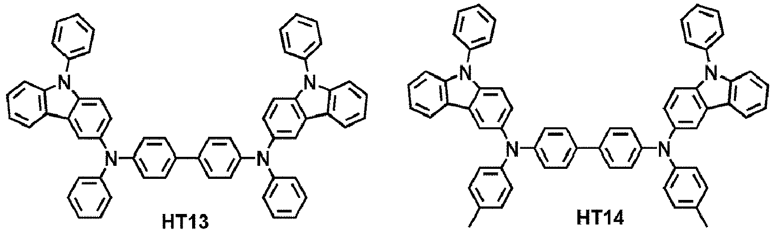

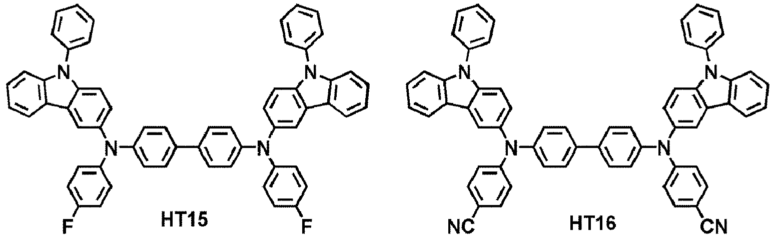

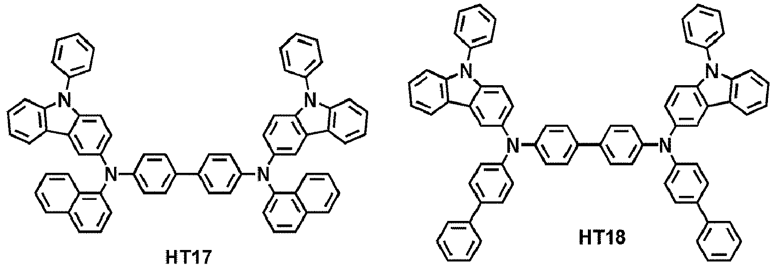

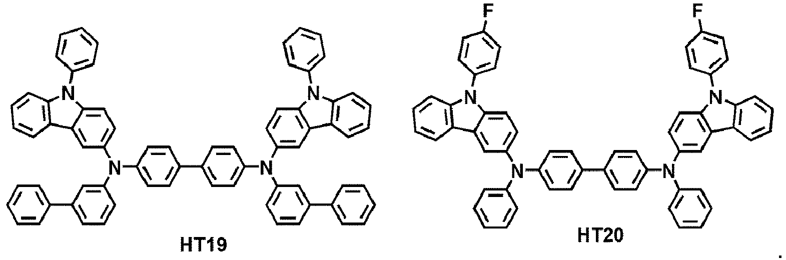

A thickness of the hole transport region may be in a range of about 100 Angstroms (Å) to about 10,000 Å, for example, about 100 Å to about 1,000 Å. When the hole transport region includes at least one of a hole injection layer and a hole transport layer, the thickness of the hole injection layer may be in a range of about 100 Å to about 10,000 Å, and for example, about 100 Å to about 1,000 Å, and the thickness of the hole transport layer may be in a range of about 50 Å to about 2,000 Å, and for example, about 100 Å to about 1,500 Å. While not wishing to be bound by theory, it is understood that when the thicknesses of the hole transport region, the hole injection layer, and the hole transport layer are within these ranges, satisfactory hole transporting characteristics may be obtained without a substantial increase in driving voltage.

The hole transport region may further include, in addition to these materials, a charge-generation material for the improvement of conductive properties. The charge-generation material may be homogeneously or non-homogeneously dispersed in the hole transport region.

The charge-generation material may be, for example, a p-dopant. The p-dopant may be one selected from a quinone derivative, a metal oxide, and a cyano group-containing compound, but embodiments of the present disclosure are not limited thereto. Non-limiting examples of the p-dopant are a quinone derivative, such as tetracyanoquinonedimethane (TCNQ) or 2,3,5,6-tetrafluoro-tetracyano-1,4-benzoquinonedimethane (F4-TCNQ); a metal oxide, such as a tungsten oxide or a molybdenium oxide; and a cyano group-containing compound, such as Compound HT-D1, but are not limited thereto:

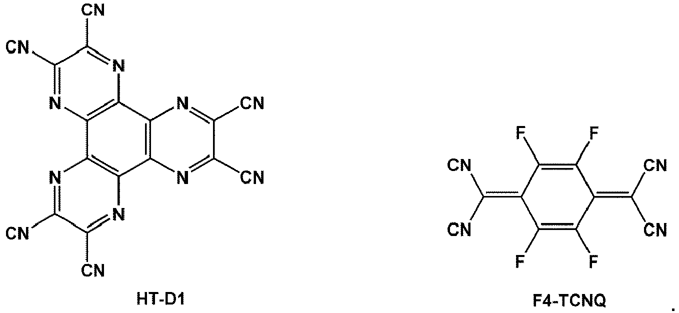

The hole transport region may include a buffer layer.

Also, the buffer layer may compensate for an optical resonance distance according to a wavelength of light emitted from the emission layer, and thus, efficiency of a formed organic light-emitting device may be improved.

Then, an emission layer may be formed on the hole transport region by vacuum deposition, spin coating, casting, LB deposition, or the like. When the emission layer is formed by vacuum deposition or spin coating, the deposition or coating conditions may be similar to those applied in forming the hole injection layer although the deposition or coating conditions may vary according to a compound that is used to form the emission layer.

Meanwhile, when the hole transport region includes an electron blocking layer, a material for the electron blocking layer may be selected from materials for the hole transport region described above and materials for a host to be explained later. However, the material for the electron blocking layer is not limited thereto. For example, when the hole transport region includes an electron blocking layer, a material for the electron blocking layer may be mCP, which will be explained later.

The emission layer may include a host and a dopant, and the dopant may include the organometallic compound represented by Formula 1.

The host may include at least one selected from TPBi, TBADN, ADN (also referred to as "DNA"), CBP, CDBP, TCP, mCP, and Compounds H50 to H52:

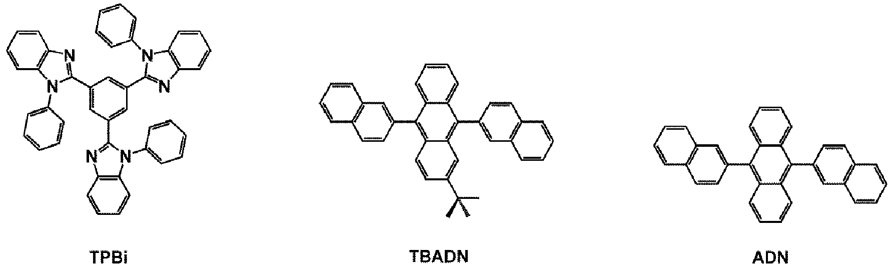

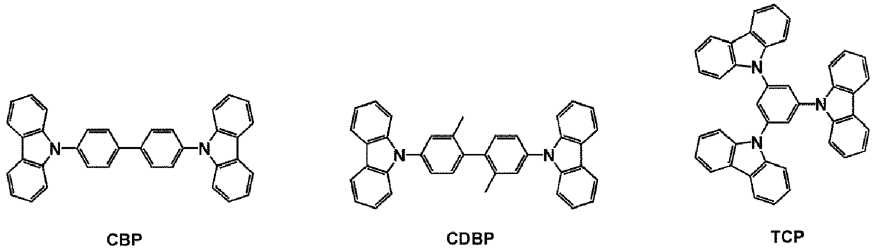

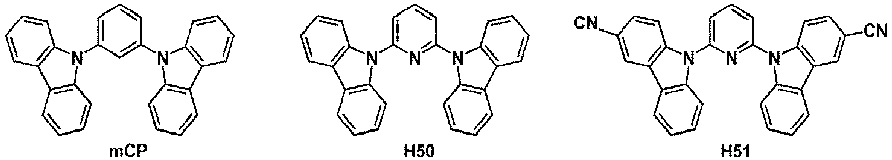

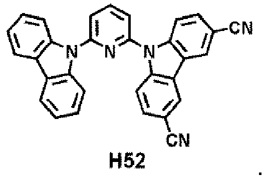

In one or more embodiments, the host may further include a compound represented by Formula 301:

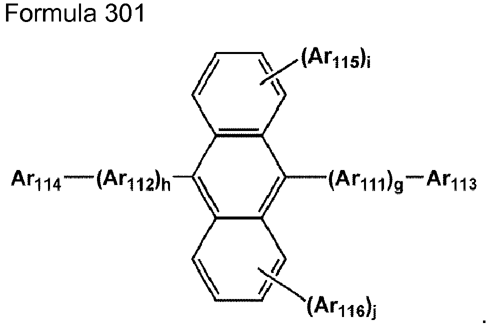

In Formula 301, Ar₁₁₁ and Ar₁₁₂ may each independently be selected from:
a phenylene group, a naphthylene group, a phenanthrenylene group, and a pyrenylene group; and
a phenylene group, a naphthylene group, a phenanthrenylene group, and a pyrenylene group, each substituted with at least one selected from a phenyl group, a naphthyl group, and an anthracenyl group.

In Formula 301, Ar₁₁₃ to Ar₁₁₆ may each independently be selected from:
a C₁-C₁₀ alkyl group, a phenyl group, a naphthyl group, a phenanthrenyl group, and a pyrenyl group; and
a phenyl group, a naphthyl group, a phenanthrenyl group, and a pyrenyl group, each substituted with at least one selected from a phenyl group, a naphthyl group, and an anthracenyl group.

In Formula 301, g, h, i, and j may each independently be an integer from 0 to 4, and may be, for example, 0, 1, or 2.

In Formula 301, Ar₁₁₃ to Ar₁₁₆ may each independently be selected from:
a C₁-C₁₀ alkyl group, the substituted with at least one selected from a phenyl group, a naphthyl group, and an anthracenyl group;
a phenyl group, a naphthyl group, an anthracenyl group, a pyrenyl, a phenanthrenyl group, and a fluorenyl group;
a phenyl group, a naphthyl group, an anthracenyl group, a pyrenyl group, a phenanthrenyl group, and a fluorenyl group, each substituted with at least one selected from deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a phenyl group, a naphthyl group, an anthracenyl group, a pyrenyl group, a phenanthrenyl group, and a fluorenyl group; and 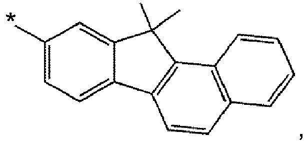
but embodiments of the present disclosure are not limited thereto.

In one or more embodiments, the host may include a compound represented by Formula 302:

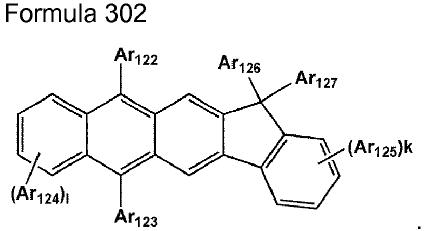

In Formula 302, Ar₁₂₂ to Ar₁₂₅ are each independently defined the same as Ar₁₁₃ in Formula 301.

In Formula 302, Ar₁₂₆ and Ar₁₂₇ may each independently be a C₁-C₁₀ alkyl group (for example, a methyl group, an ethyl group, or a propyl group).

In Formula 302, k and I may each independently be an integer from 0 to 4. For example, k and I may be 0, 1, or 2.

When the organic light-emitting device is a full-color organic light-emitting device, the emission layer may be patterned into a red emission layer, a green emission layer, and a blue emission layer. In one or more embodiments, due to a stacked structure including a red emission layer, a green emission layer, and/or a blue emission layer, the emission layer may emit white light.

When the emission layer includes a host and a dopant, an amount of the dopant may be in a range of about 0.01 parts by weight to about 30 parts by weight based on 100 parts by weight of the host, but embodiments of the present disclosure are not limited thereto.

A thickness of the emission layer may be in a range of about 100 Å to about 1,000 Å, for example, about 200 Å to about 600 Å. While not wishing to be bound by theory, it is understood that when the thickness of the emission layer is within this range, excellent light-emission characteristics may be obtained without a substantial increase in driving voltage.

Then, an electron transport region may be disposed on the emission layer.

The electron transport region may include a hole blocking layer, an electron transport layer, an electron injection layer, or any combination thereof.

For example, the electron transport region may have a hole blocking layer/electron transport layer/electron injection layer structure or an electron transport layer/electron injection layer structure, but the structure of the electron transport region is not limited thereto. The electron transport layer may have a single-layered structure or a multi-layered structure including two or more different materials.

Conditions for forming the hole blocking layer, the electron transport layer, and the electron injection layer which constitute the electron transport region may be understood by referring to the conditions for forming the hole injection layer.

When the electron transport region includes a hole blocking layer, the hole blocking layer may include, for example, at least one of BCP, BPhen, and BAlq but embodiments of the present disclosure are not limited thereto:

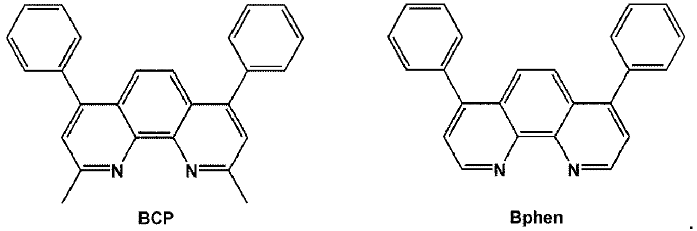

A thickness of the hole blocking layer may be in a range of about 20 Å to about 1,000 Å, for example, about 30 Å to about 300 Å. While not wishing to be bound by theory, it is understood that when the thickness of the hole blocking layer is within these ranges, the hole blocking layer may have improved hole blocking ability without a substantial increase in driving voltage.

The electron transport layer may further include at least one selected from BCP, BPhen, Alq₃, BAlq, TAZ, and NTAZ:

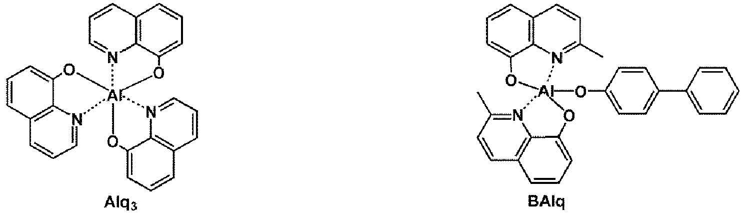

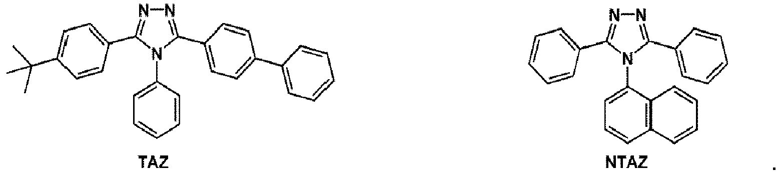

In one or more embodiments, the electron transport layer may include at least one of ET1 and ET25, but are not limited thereto:

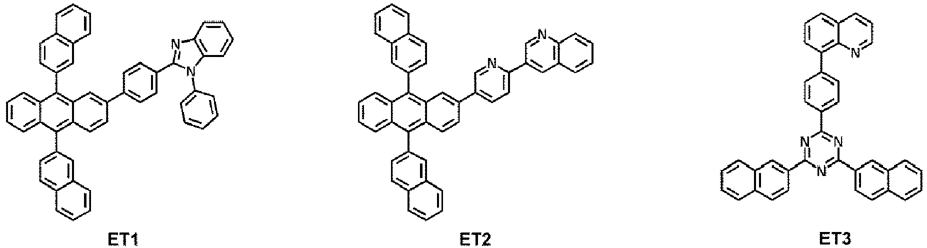

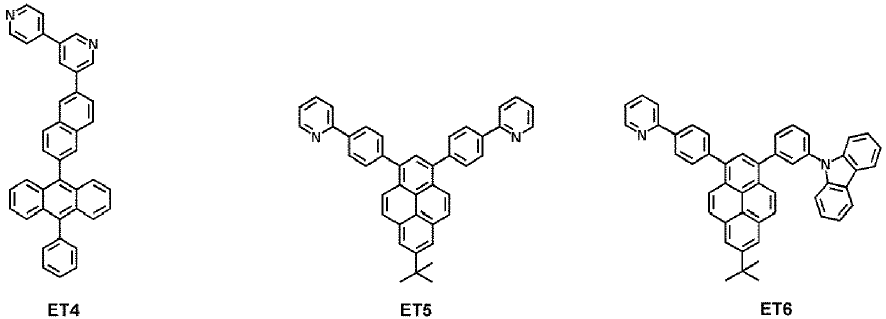

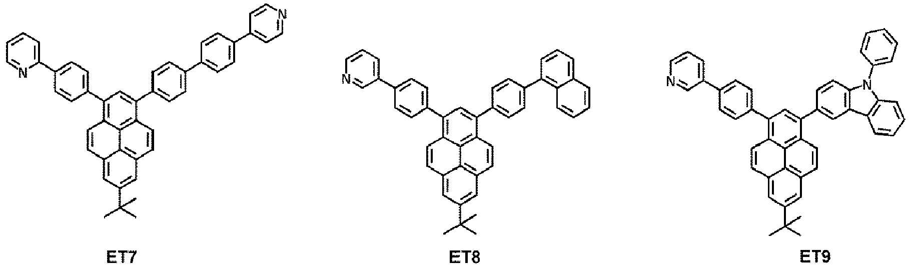

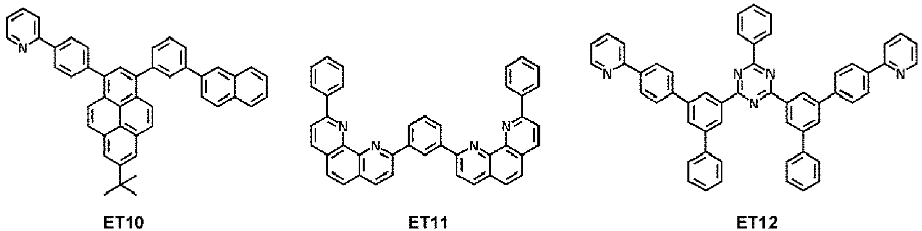

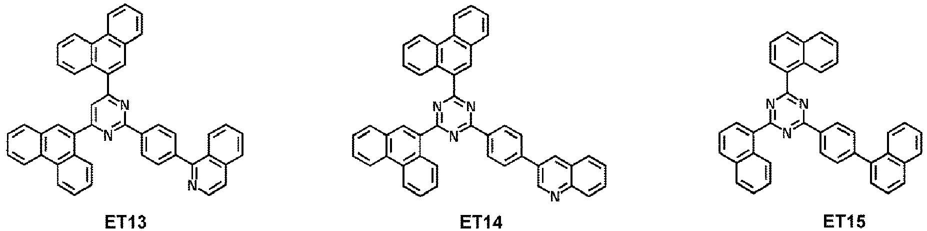

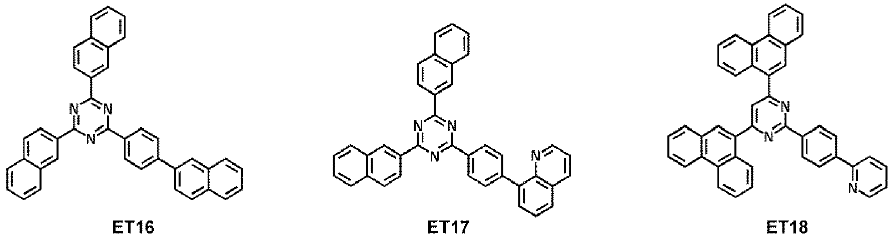

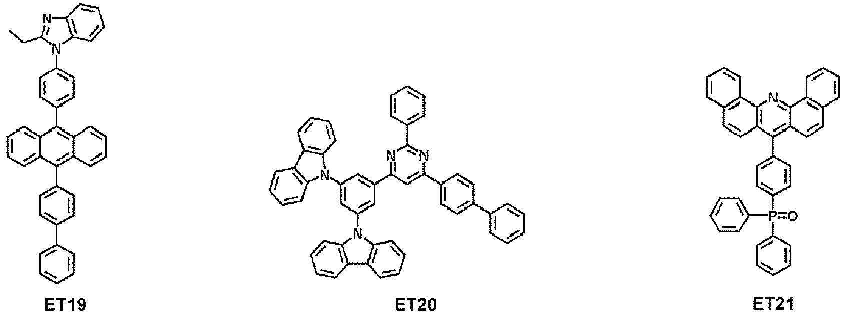

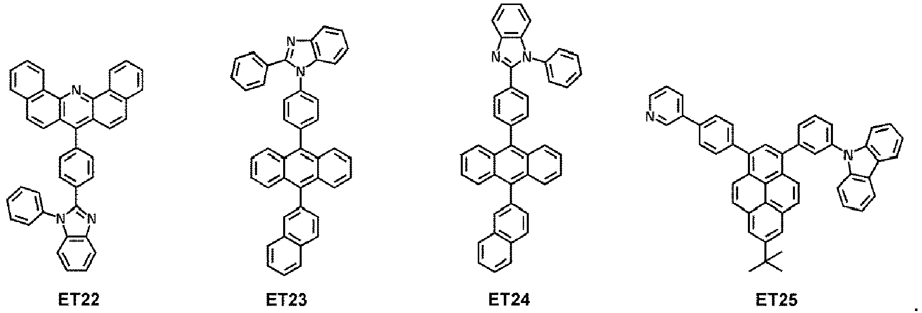

A thickness of the electron transport layer may be in a range of about 100 Å to about 1,000 Å, for example, about 150 Å to about 500 Å. While not wishing to be bound by theory, it is understood that when the thickness of the electron transport layer is within the range described above, the electron transport layer may have satisfactory electron transport characteristics without a substantial increase in driving voltage.

Also, the electron transport layer may further include, in addition to the materials described above, a metal-containing material.

The metal-containing material may include a Li complex. The Li complex may include, for example, Compound ET-D1 (lithium 8-hydroxyquinolate, LiQ) or ET-D2:

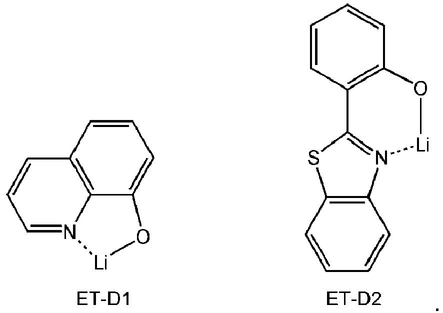

The electron transport region may include an electron injection layer that promotes flow of electrons from the second electrode 19 thereinto.

The electron injection layer may include at least one selected from LiF, NaCl, CsF, Li₂O, and BaO.

A thickness of the electron injection layer may be in a range of about 1 Å to about 100 Å, for example, about 3 Å to about 90 Å. While not wishing to be bound by theory, it is understood that when the thickness of the electron injection layer is within the range described above, the electron injection layer may have satisfactory electron injection characteristics without a substantial increase in driving voltage.

The second electrode 19 is disposed on the organic layer 15. The second electrode 19 may be a cathode. A material for forming the second electrode 19 may be selected from metal, an alloy, an electrically conductive compound, and a combination thereof, which have a relatively low work function. For example, lithium (Li), magnesium (Mg), aluminum (Al), aluminum-lithium (Al-Li), calcium (Ca), magnesium-indium (Mg-In), or magnesium-silver (Mg-Ag) may be used as a material for forming the second electrode 19. In one or more embodiments, to manufacture a top-emission type light-emitting device, a transmissive electrode formed using ITO or IZO may be used as the second electrode 19.

Hereinbefore, the organic light-emitting device has been described with reference to the FIGURE, but embodiments of the present disclosure are not limited thereto.

Another aspect of the present disclosure provides a diagnostic composition including at least one organometallic compound represented by Formula 1.

The organometallic compound represented by Formula 1 provides high luminescent efficiency. Accordingly, a diagnostic composition including the organometallic compound may have a high diagnostic efficiency.

The diagnostic composition may be used in various applications including a diagnosis kit, a diagnosis reagent, a biosensor, and a biomarker.

The term "C₁-C₆₀ alkyl group" as used herein refers to a linear or branched saturated aliphatic hydrocarbon monovalent group having 1 to 60 carbon atoms, and non-limiting examples thereof include a methyl group, an ethyl group, a propyl group, an iso-butyl group, a sec-butyl group, a tert-butyl group, a pentyl group, an iso-amyl group, and a hexyl group. The term "C₁-C₆₀ alkylene group" as used herein refers to a divalent group having the same structure as the C₁-C₆₀ alkyl group.

The term "C₁-C₆₀ alkoxy group" as used herein refers to a monovalent group represented by -OA₁₀₁ (wherein A₁₀₁ is the C₁-C₆₀ alkyl group), and non-limiting examples thereof include a methoxy group, an ethoxy group, and an iso-propyloxy group.

The term "C₂-C₆₀ alkenyl group" as used herein refers to a hydrocarbon group formed by including at least one carbon-carbon double bond in the middle or at the terminus of the C₂-C₆₀ alkyl group, and examples thereof include an ethenyl group, a propenyl group, and a butenyl group. The term "C₂-C₆₀ alkenylene group" as used herein refers to a divalent group having the same structure as the C₂-C₆₀ alkenyl group.

The term "C₂-C₆₀ alkynyl group" as used herein refers to a hydrocarbon group formed by including at least one carbon-carbon triple bond in the middle or at the terminus of the C₂-C₆₀ alkyl group, and examples thereof include an ethynyl group, and a propynyl group. The term "C₂-C₆₀ alkynylene group" as used herein refers to a divalent group having the same structure as the C₂-C₆₀ alkynyl group.

The term "C₃-C₁₀ cycloalkyl group" as used herein refers to a monovalent saturated hydrocarbon monocyclic group having 3 to 10 carbon atoms, and non-limiting examples thereof include a cyclopropyl group, a cyclobutyl group, a cyclopentyl group, a cyclohexyl group, and a cycloheptyl group. The term "C₃-C₁₀ cycloalkylene group" as used herein refers to a divalent group having the same structure as the C₃-C₁₀ cycloalkyl group.

The term "C₁-C₁₀ heterocycloalkyl group" as used herein refers to a monovalent saturated monocyclic group having at least one heteroatom selected from N, O, P, Si and S as a ring-forming atom and 1 to 10 carbon atoms, and non-limiting examples thereof include a tetrahydrofuranyl group, and a tetrahydrothiophenyl group. The term "C₁-C₁₀ heterocycloalkylene group" as used herein refers to a divalent group having the same structure as the C₁-C₁₀ heterocycloalkyl group.

The term "C₃-C₁₀ cycloalkenyl group" as used herein refers to a monovalent monocyclic group that has 3 to 10 carbon atoms and at least one carbon-carbon double bond in the ring thereof and no aromaticity, and non-limiting examples thereof include a cyclopentenyl group, a cyclohexenyl group, and a cycloheptenyl group. The term "C₃-C₁₀ cycloalkenylene group" as used herein refers to a divalent group having the same structure as the C₃-C₁₀ cycloalkenyl group.

The term "C₁-C₁₀ heterocycloalkenyl group" as used herein refers to a monovalent monocyclic group that has at least one heteroatom selected from N, O, P, Si, and S as a ring-forming atom, 1 to 10 carbon atoms, and at least one carbon-carbon double bond in its ring. Examples of the C₁-C₁₀ heterocycloalkenyl group are a 2,3-dihydrofuranyl group, and a 2,3-dihydrothiophenyl group. The term "C₁-C₁₀ heterocycloalkenylene group" as used herein refers to a divalent group having the same structure as the C₁-C₁₀ heterocycloalkenyl group.

The term "C₆-C₆₀ aryl group" as used herein refers to a monovalent group having a carbocyclic aromatic system having 6 to 60 carbon atoms, and the term "C₆-C₆₀ arylene group" as used herein refers to a divalent group having a carbocyclic aromatic system having 6 to 60 carbon atoms. Non-limiting examples of the C₆-C₆₀ aryl group include a phenyl group, a naphthyl group, an anthracenyl group, a phenanthrenyl group, a pyrenyl group, and a chrysenyl group. When the C₆-C₆₀ aryl group and the C₆-C₆₀ arylene group each include two or more rings, the rings may be fused to each other.

The term "C₁-C₆₀ heteroaryl group" as used herein refers to a monovalent group having a carbocyclic aromatic system that has at least one heteroatom selected from N, O, P, Si, and S as a ring-forming atom, and 1 to 60 carbon atoms. The term "C₁-C₆₀ heteroarylene group" as used herein refers to a divalent group having a carbocyclic aromatic system that has at least one heteroatom selected from N, O, P, and S as a ring-forming atom, and 1 to 60 carbon atoms. Non-limiting examples of the C₁-C₆₀ heteroaryl group include a pyridinyl group, a pyrimidinyl group, a pyrazinyl group, a pyridazinyl group, a triazinyl group, a quinolinyl group, and an isoquinolinyl group. When the C₁-C₆₀ heteroaryl group and the C₁-C₆₀ heteroarylene group each include two or more rings, the rings may be fused to each other.

The term "C₆-C₆₀ aryloxy group" used herein indicates -OA₁₀₂ (wherein A₁₀₂ is the C₆-C₆₀ aryl group), a C₆-C₆₀ arylthio group used herein indicates -SA₁₀₃ (wherein A₁₀₃ is the C₆-C₆₀ aryl group), and the term "C₇-C₆₀ arylalkyl group" as used herein indicates -A₁₀₄A₁₀₅ (wherein A₁₀₅ is the C₆-C₅₉ aryl group and A₁₀₄ is the C₁-C₅₃ alkylene group).

The term "C₁-C₆₀ heteroaryloxy group" as used herein refers to -OA₁₀₆ (wherein A₁₀₆ is the C₂-C₆₀ heteroaryl group), the term "C₁-C₆₀ heteroarylthio group" as used herein indicates -SA₁₀₇ (wherein A₁₀₇ is the C₁-C₆₀ heteroaryl group), and the term "C₂-C₆₀ heteroarylalkyl group" as used herein refers to -A₁₀₈A₁₀₉ (A₁₀₉ is a C₁-C₅₉ heteroaryl group, and A₁₀₈ is a C₁-C₅₉ alkylene group).

The term "monovalent non-aromatic condensed polycyclic group" as used herein refers to a monovalent group (for example, having 8 to 60 carbon atoms) having two or more rings condensed to each other, only carbon atoms as ring-forming atoms, and no aromaticity in its entire molecular structure. Examples of the monovalent non-aromatic condensed polycyclic group include a fluorenyl group. The term "divalent non-aromatic condensed polycyclic group" as used herein refers to a divalent group having the same structure as the monovalent non-aromatic condensed polycyclic group.

The term "monovalent non-aromatic condensed heteropolycyclic group" as used herein refers to a monovalent group (for example, having 2 to 60 carbon atoms) having two or more rings condensed to each other, a heteroatom selected from N, O, P, Si, and S, other than carbon atoms, as a ring-forming atom, and no aromaticity in its entire molecular structure. Non-limiting examples of the monovalent non-aromatic condensed heteropolycyclic group include a carbazolyl group. The term "divalent non-aromatic condensed heteropolycyclic group" as used herein refers to a divalent group having the same structure as the monovalent non-aromatic condensed heteropolycyclic group.

The term "C₅-C₃₀ carbocyclic group" as used herein refers to a saturated or unsaturated cyclic group having, as a ring-forming atom, 5 to 30 carbon atoms only. The C₅-C₃₀ carbocyclic group may be a monocyclic group or a polycyclic group.

The term "C₁-C₃₀ heterocyclic group" as used herein refers to a saturated or unsaturated cyclic group having, as a ring-forming atom, at least one heteroatom selected from N, O, Si, P, and S other than 1 to 30 carbon atoms. The C₁-C₃₀ heterocyclic group may be a monocyclic group or a polycyclic group.

At least one substituent of the substituted C₅-C₃₀ carbocyclic group, the substituted C₂-C₃₀ heterocyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₇-C₆₀ arylalkyl group, the substituted C₁-C₆₀ heteroaryl group, the substituted C₁-C₆₀ heteroaryloxy group, the substituted C₁-C₆₀ heteroarylthio group, the substituted C₂-C₆₀ heteroarylalkyl group, the substituted monovalent non-aromatic condensed polycyclic group, and the substituted monovalent non-aromatic condensed heteropolycyclic group may be selected from:
deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, a monovalent non-aromatic condensed heteropolycyclic group, -N(Q₁₁)(Q₁₂),-Si(Q₁₃)(Q₁₄)(Q₁₅), -B(Q₁₆)(Q₁₇), and -P(=O)(Q₁₈)(Q₁₉);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, and a monovalent non-aromatic condensed heteropolycyclic group, each substituted with at least one selected from deuterium, -F, -Cl, -Br, -I, -CD₃,-CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, a monovalent non-aromatic condensed heteropolycyclic group, -N(Q₂₁)(Q₂₂),-Si(Q₂₃)(Q₂₄)(Q₂₅), -B(Q₂₆)(Q₂₇), and -P(=O)(Q₂₈)(Q₂₉); and
-N(Q₃₁)(Q₃₂), -Si(Q₃₃)(Q₃₄)(Q₃₅), -B(Q₃₆)(Q₃₇), and -P(=O)(Q₃₈)(Q₃₉), and
Q₁ to Q₉, Q₁₁ to Q₁₉, Q₂₁ to Q₂₉, and Q₃₁ to Q₃₉ may each independently be selected from hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryl group substituted with at least one selected from a C₁-C₆₀ alkyl group, and a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, and a monovalent non-aromatic condensed heteropolycyclic group.

Hereinafter, a compound and an organic light-emitting device according to embodiments are described in detail with reference to Synthesis Example and Examples. However, the organic light-emitting device is not limited thereto. The wording "B was used instead of A" used in describing Synthesis Examples means that an amount of A used was identical to an amount of B used, in terms of a molar equivalent.

### EXAMPLES

### Synthesis Example 1 : Synthesis of Compound 1

Compound 1 was synthesized according to Reaction Scheme 1.

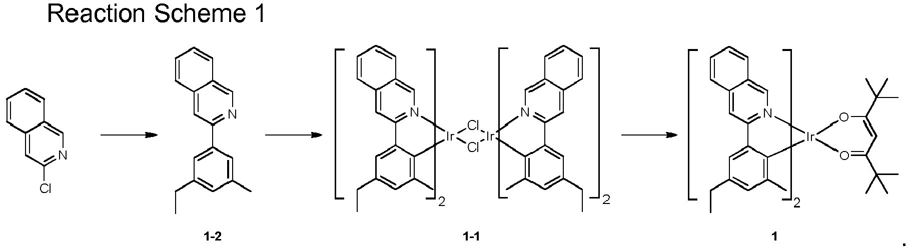

### Synthesis of Intermediate 1-2

3-chloroisoquinoline (3.40 grams (g), 20.76 millimoles, mmol), (3-ethyl-5-methylphenyl)boronic acid (5.11 g, 31.14 mmol), Pd(PPh₃)₄ (1.92 g, 1.66 mmol), and K₂CO₃ (7.17 g, 51.90 mmol) were mixed with 60 milliliters (mL) of tetrahydrofuran (THF) and 30 mL of distilled water, and the mixed solution was stirred under reflux for 18 hours. After the temperature of the mixed solution was cooled to room temperature, an extraction was performed by using methylene chloride (MC). The organic layer extracted therefrom was then treated with anhydrous magnesium sulfate (MgSO4) to remove the solvent. The filtrate obtained by filtering the resulting mixed solution was reduced under pressure to produce a residue, and the residue thus obtained was separated and purified by column chromatography with EA:Hexane = 1:10 as an eluent, thereby obtaining 4.78 g (93%) of Intermediate 1-2.
MALDI-TOFMS (m/z): C18H17N (M+) 247.14

### Synthesis of Intermediate 1-1

Intermediate 1-2 (4.78 g, 19.3 mmol) and iridium chloride (3.02 g, 8.58 mmol) were mixed with 45 mL of ethoxyethanol and 15 mL of distilled water. The mixed solution was then stirred under reflux for 24 hours and cooled to room temperature. The resulting solid was separated by filtration, and thoroughly washed with water/methanol/hexane in the stated order. The solid product thus obtained was dried in a vacuum oven, thereby obtaining Intermediate 1-1 (3.96 g, 57%).

### Synthesis of Compound 1

Intermediate 1-1 (1.17 g, 0.81 mmol), 2,2,6,6-tetramethylheptane-3,5-dione (1.49 g, 8.14 mmol), and Na₂CO₃ (0.83 g, 8.14 mmol) were mixed with 15 mL of ethoxyethanol, and the mixed solution was stirred for 24 hours to allow the reaction to proceed. The resulting mixture was filtered to produce a solid product, and the solid product was thoroughly washed with ethanol and hexane and subjected to column chromatography with dichloromethane:n-hexane=1:1 (volume to volume, v/v) as an eluent, thereby obtaining Compound 1 (0.45 g, 32%). Compound 1 was then identified by Mass and HPLC.
HRMS (MALDI) calcd for C47H51IrN2O2: m/z 868.3580, Found: 868.3577.

### Synthesis Example 2: Synthesis of Compound 7

Compound 7 was synthesized according to Reaction Scheme 2.

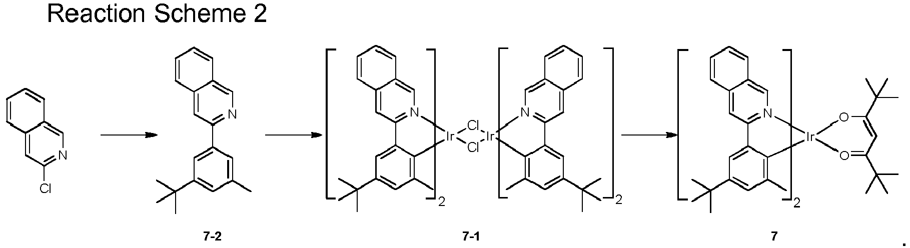

### Synthesis of Intermediate 7-2

Intermediate 7-2 was synthesized in the same manner as Intermediate 1-2 of Synthesis Example 1, except that (3-(tert-butyl)-5-methylphenyl)boronic acid was used instead of (3-ethyl-5-methylphenyl)boronic acid).

### Synthesis of Intermediate 7-1

Intermediate 7-1 was synthesized in the same manner as Intermediate 1-1 of Synthesis Example 1, except that Intermediate 7-2 was used instead of Intermediate 1-2.

### Synthesis of Compound 7

Compound 7 was synthesized in the same manner as Compound 1 of Synthesis Example 1, except that Intermediate 7-1 was used instead of Intermediate 1-1. Compound 7 was then identified by Mass and HPLC.
HRMS (MALDI) calcd for C51H59IrN2O2: m/z 924.4206, Found: 924.4207.

### Synthesis Example 3 : Synthesis of Compound 28

Compound 28 was synthesized according to Reaction Scheme 3.

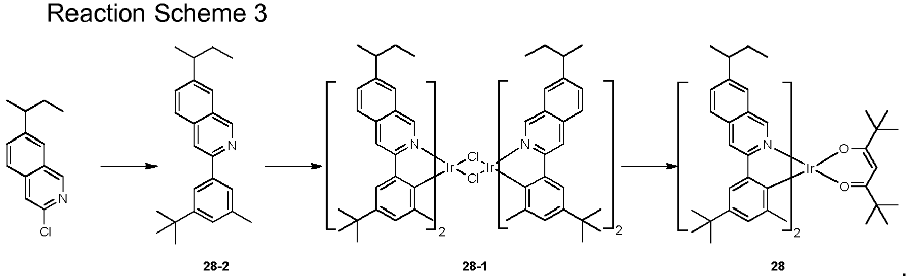

### Synthesis of Intermediate 28-2

Intermediate 28-2 was synthesized in the same manner as Intermediate 7-2 of Synthesis Example 2, except that 7-(sec-butyl)-3-chloroisoquinoline was used instead of 3-chloroisoquinoline.

### Synthesis of Intermediate 28-1

Intermediate 28-1 was synthesized in the same manner as Intermediate 1-1 of Synthesis Example 1, except that Intermediate 28-2 was used instead of Intermediate 1-2.

### Synthesis of Compound 28

Compound 28 was synthesized in the same manner as Compound 1 of Synthesis Example 1, except that Intermediate 28-1 was used instead of Intermediate 1-1. Compound 28 was then identified by Mass and HPLC.
HRMS (MALDI) calcd for C59H75IrN2O2: m/z 1036.5458, Found: 1036.5455.

### Synthesis Example 4 : Synthesis of Compound 41

Compound 41 was synthesized according to Reaction Scheme 4.

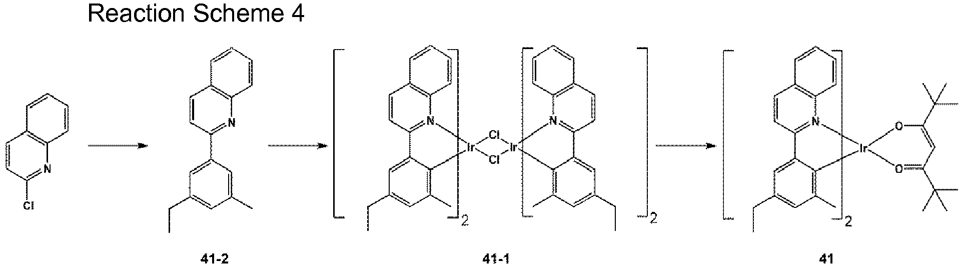

### Synthesis of Intermediate 41-2

Intermediate 41-2 was synthesized in the same manner as Intermediate 1-2 of Synthesis Example 1, except that 2-chloroquinoline was used instead of 3-chloroisoquinoline.

### Synthesis of Intermediate 41-1

Intermediate 41-1 was synthesized in the same manner as Intermediate 1-1 of Synthesis Example 1, except that Intermediate 41-2 was used instead of Intermediate 1-2.

### Synthesis of Compound 41

Compound 41 was synthesized in the same manner as Compound 1 of Synthesis Example 1, except that Intermediate 41-1 was used instead of Intermediate 1-1. Compound 41 was then identified by Mass and HPLC.
HRMS (MALDI) calcd for C47H51IrN2O2: m/z 868.3580, Found: 868.3578.

### Synthesis Example 5: Synthesis of Compound 47

Compound 47 was synthesized according to Reaction Scheme 5.

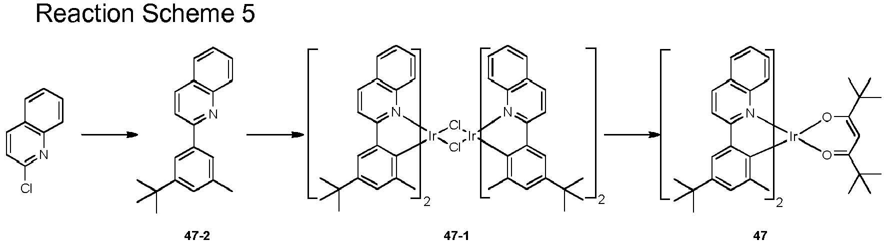

### Synthesis of Intermediate 47-2

Intermediate 47-2 was synthesized in the same manner as Intermediate 41-2 of Synthesis Example 4, except that (3-(tert-butyl)-5-methylphenyl)boronic acid was used instead of (3-ethyl-5-methylphenyl)boronic acid).

### Synthesis of Intermediate 47-1

Intermediate 47-1 was synthesized in the same manner as Intermediate 1-1 of Synthesis Example 1, except that Intermediate 47-2 was used instead of Intermediate 1-2.

### Synthesis of Compound 47

Compound 47 was synthesized in the same manner as Compound 1 of Synthesis Example 1, except that Intermediate 47-1 was used instead of Intermediate 1-1. Compound 47 was then identified by Mass and HPLC.
HRMS (MALDI) calcd for C51H59IrN2O2: m/z 924.4206, Found: 924.4203.

### Synthesis Example 6 : Synthesis of Compound 69

Compound 69 was synthesized according to Reaction Scheme 6.

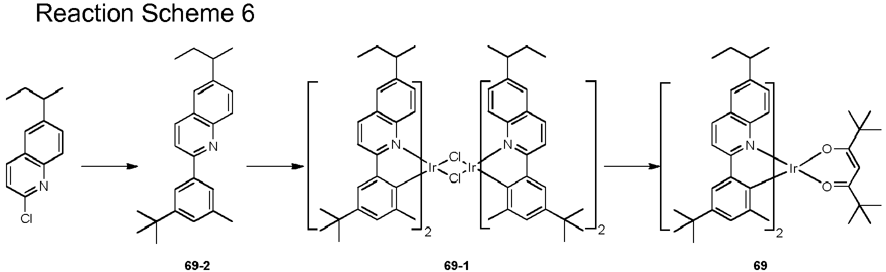

### Synthesis of Intermediate 69-2

Intermediate 69-2 was synthesized in the same manner as Intermediate 47-2 of Synthesis Example 5, except that 6-(sec-butyl)-2-chloroquinoline was used instead of 2-chloroquinoline.

### Synthesis of Intermediate 69-1

Intermediate 69-1 was synthesized in the same manner as Intermediate 1-1 of Synthesis Example 1, except that Intermediate 69-2 was used instead of Intermediate 1-2.

### Synthesis of Compound 69

Compound 69 was synthesized in the same manner as Compound 1 of Synthesis Example 1, except that Intermediate 69-1 was used instead of Intermediate 1-1. Compound 69 was then identified by Mass and HPLC.
HRMS (MALDI) calcd for C59H75IrN2O2: m/z 1036.5458, Found: 1036.5456.

### Example 1

As an anode, a glass substrate, on which ITO/Ag/ITO (70 Å/1,000 Å/70 Å) was deposited, was cut to a size of 50 mm x 50 mm x 0.5 mm (mm = millimeter), sonicated with iso-propyl alcohol and pure water each for 5 minutes, and cleaned by exposure to ultraviolet rays and ozone for 30 minutes. Then, the glass substrate was provided to a vacuum deposition apparatus.

2-TNATA was vacuum-deposited on the anode of the glass substrate to form a hole injection layer having a thickness of 600 Å, and 4,4'-bis[N-(1-naphthyl)-N-phenylamino]biphenyl (NPB) was vacuum-deposited on the hole injection layer to form a hole transport layer having a thickness of 1,350 Å.

Next, CBP (host) and Compound 1 (dopant) were co-deposited on the hole transport layer at a weight ratio of 98 : 2 to form an emission layer having a thickness of 400 Å.

Afterwards, BCP was vacuum-deposited on the emission layer to form a hole blocking layer having a thickness of 50 Å, Alq₃ was vacuum-deposited on the hole blocking layer to form an electron transport layer having a thickness of 350 Å, LiF was deposited on the electron transport layer to form an electron injection layer having a thickness of 10 Å, and Mg and Ag were co-deposited on the electron injection layer at a weight ratio of 90:10 to form a cathode having a thickness of 120 Å, thereby completing an organic light-emitting device (emitting red light).

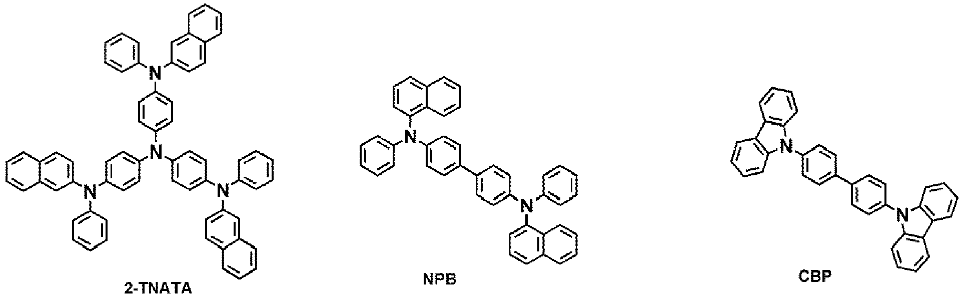

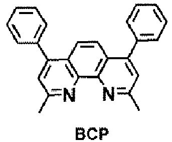

Examples 2 to 6 and Comparative Examples 1 to 7

Organic light-emitting devices were manufactured in the same manner as in Example 1, except that in forming an emission layer, for use as a dopant, corresponding compounds shown in Table 2 were used instead of Compound 1.

### Evaluation Example 2: Evaluation on characteristics of organic light-emitting devices.

The driving voltage, maximum efficiency (Max Cd/A), roll-off ratio, full width at half maximum and maximum emission wavelength of main peaks in EL spectra, and lifespan (T₉₇) of the organic light-emitting devices manufactured according to Examples 1 to 6 and Comparative Examples 1 to 7 were evaluated, and results thereof are shown in Table 3. Here, devices used in the evaluation are a current-voltage meter (Keithley 2400) and a luminance meter (Minolta Cs-1000A), and the lifespan (T₉₇) indicates an amount of time that elapsed when luminance was 97% of initial luminance (100%). The roll-off ratio was calculated by Equation 20. $Roll off = \left\{1-\left(efficiency \left(at 3,500 nit\right)/maximum luminance efficiency\right)\right\} \times 100 %$

**Table 2**

| | Dopant in emission layer | Driving voltage (V) | Max Cd/A (%) | Roll-Off (%) | LT97 (hr) |
|---|---|---|---|---|---|
| Example 1 | Compound 1 | 4.65 | 48.5 | 16 | 250 |
| Example 2 | Compound 7 | 4.62 | 52.1 | 14 | 300 |
| Example 3 | Compound 28 | 4.72 | 52.4 | 14 | 250 |
| Example 4 | Compound 41 | 4.78 | 33.2 | 11 | 300 |
| Example 5 | Compound 47 | 4.72 | 35.8 | 11 | 330 |
| Example 6 | Compound 69 | 5.05 | 32.1 | 13 | 300 |
| Comparative Example 1 | Compound A | 5.49 | 20.4 | 28 | 50 |
| Comparative Example 2 | Compound B | 5.42 | 15.5 | 19 | 160 |
| Comparative Example 3 | Compound C | 5.35 | 20.2 | 16 | 280 |
| Comparative Example 4 | Compound D | 5.38 | 18.7 | 17 | 150 |
| Comparative Example 5 | Compound E | 4.86 | 28.1 | 16 | 215 |
| Comparative Example 6 | Compound F | 5.02 | 17,7 | 23 | 190 |
| Comparative Example 7 | Compound G | 4.83 | 20.1 | 12 | 230 |

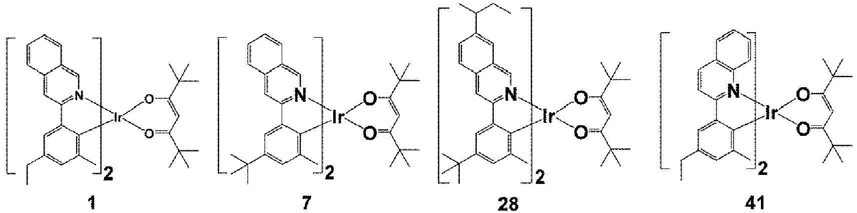

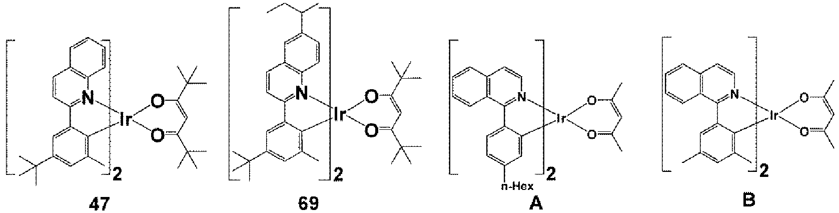

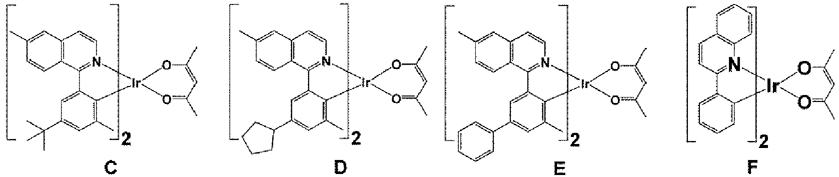

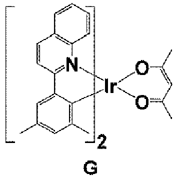

In Compound A, "n-Hex" indicates an n-hexyl group.

Referring to Table 2, it was confirmed that the organic light-emitting devices of Examples 1 to 6 emit red light and have improved driving voltage, external quantum efficiency, roll-off ratios, and lifespan characteristics, compared to the organic light-emitting devices of Comparative Examples 1 to 7.

According to the one or more embodiments, an organometallic compound may have excellent electric characteristics and thermal stability, and thus, an organic light-emitting device including the organometallic compound may also have excellent driving voltage, emission efficiency, quantum luminance efficiency, roll-off ratio, and lifespan characteristics.

It should be understood that embodiments described herein should be considered in a descriptive sense only and not for purposes of limitation. Descriptions of features or aspects within each embodiment should typically be considered as available for other similar features or aspects in other embodiments.

While one or more embodiments have been described with reference to the figures, it will be understood by those of ordinary skill in the art that various changes in form and details may be made therein without departing from the scope of the present disclosure as defined by the following claims.

## Claims

1. An organometallic compound represented by Formula 1:
Formula 1 M(L₁)ₙ₁(L₂)ₙ₂,
wherein, in Formula 1,
M is a transition metal,
L₁ is a ligand represented by Formula 2 or Formula 3,
n1 is 1, 2, or 3, wherein, when n1 is two or more, two or more groups L₁ are identical to or different from each other,
L₂ is selected from a monodentate ligand, a bidentate ligand, a tridentate ligand, and a tetradentate ligand,
n2 is 0, 1, 2, 3, or 4, wherein, when n2 is two or more, two or more groups L₂ are identical to or different from each other,
L₁ and L₂ are different from each other, 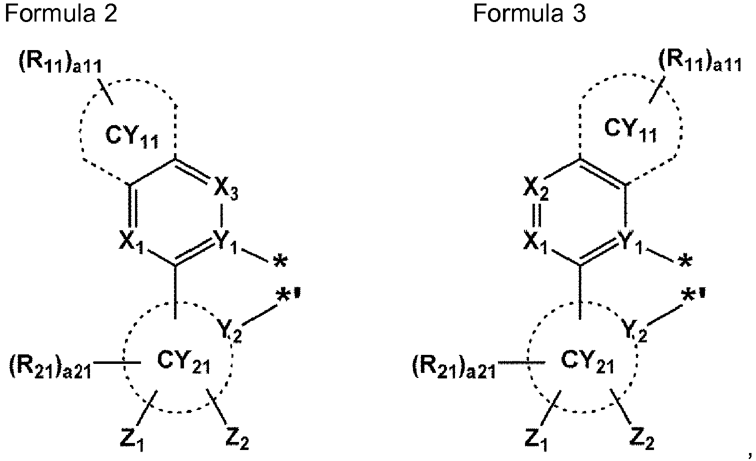
wherein, in Formulae 2 and 3,
CY₁₁ and CY₂₁ are each independently selected from a C₅-C₃₀ carbocyclic group and a C₁-C₃₀ heterocyclic group,
Y₁ and Y₂ are each independently selected from C and N,
X₁ is N or C(R₁), X₂ is N or C(R₂), X₃ is N or C(R₃),
R₁ to R₃, R₁₁, and R₂₁ are each independently selected from hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₇-C₆₀ arylalkyl group, a substituted or unsubstituted C₁-C₆₀ heteroaryl group, a substituted or unsubstituted C₁-C₆₀ heteroaryloxy group, a substituted or unsubstituted C₁-C₆₀ heteroarylthio group, a substituted or unsubstituted C₂-C₆₀ heteroarylalkyl group, a substituted or unsubstituted monovalent non-aromatic condensed polycyclic group, a substituted or unsubstituted monovalent non-aromatic condensed heteropolycyclic group, -N(Q₁)(Q₂), - Si(Q₃)(Q₄)(Q₅), -Ge(Q₃)(Q₄)(Q₅), -B(Q₆)(Q₇), and -P(=O)(Q₈)(Q₉),
a11 is an integer from 0 to 10, wherein, when a11 is two or more, two or more groups R₁₁ are identical to or different from each other,
a21 is an integer from 0 to 8, wherein, when a21 is two or more, two or more groups R₂₁ are identical to or different from each other,
Z₁ and Z₂ are each independently selected a C₁-C₆₀ alkyl group and a deuterium-containing C₁-C₆₀ alkyl group,
Z₁ and Z₂ are different from each other,
* and *' each indicate a binding site to M in Formula 1,
at least one substituent of the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₇-C₆₀ arylalkyl group, the substituted C₁-C₆₀ heteroaryl group, the substituted C₁-C₆₀ heteroaryloxy group, the substituted C₁-C₆₀ heteroarylthio group, the substituted C₂-C₆₀ heteroarylalkyl group, the substituted monovalent non-aromatic condensed polycyclic group, and the substituted monovalent non-aromatic condensed heteropolycyclic group is selected from:
deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one selected from deuterium, -F, -Cl, - Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, a monovalent non-aromatic condensed heteropolycyclic group, -N(Q₁₁)(Q₁₂), - Si(Q₁₃)(Q₁₄)(Q₁₅), -Ge(Q₁₃)(Q₁₄)(Q₁₅), -B(Q₁₆)(Q₁₇), and -P(=O)(Q₁₈)(Q₁₉);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, and a monovalent non-aromatic condensed heteropolycyclic group, each substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, a monovalent non-aromatic condensed heteropolycyclic group, -N(Q₂₁)(Q₂₂), -Si(Q₂₃)(Q₂₄)(Q₂₅), -Ge(Q₂₃)(Q₂₄)(Q₂₅), - B(Q₂₆)(Q₂₇), and -P(=O)(Q₂₈)(Q₂₉); and
-N(Q₃₁)(Q₃₂), -Si(Q₃₃)(Q₃₄)(Q₃₅), -Ge(Q₃₃)(Q₃₄)(Q₃₅), -B(Q₃₆)(Q₃₇), and - P(=O)(Q₃₈)(Q₃₉), and
Q₁ to Q₉, Q₁₁ to Q₁₉, Q₂₁ to Q₂₉, and Q₃₁ to Q₃₉ are each independently selected from hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryl group substituted with at least one selected from a C₁-C₆₀ alkyl group, and a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, and a monovalent non-aromatic condensed heteropolycyclic group.

2. The organometallic compound of claim 1, wherein
M is Ir or Os, and
n1+n2 is 3 or 4.

3. The organometallic compound of claims 1 or 2, wherein
in Formulae 2 and 3,
ring CY₁₁ and ring CY₂₁ are each independently selected from a benzene group, a naphthalene group, an anthracene group, a phenanthrene group, a triphenylene group, a pyrene group, a chrysene group, a cyclopentadiene group, a 1,2,3,4-tetrahydronaphthalene group, a thiophene group, a furan group, a selenophene group, an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isooxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, and a 5,6,7,8-tetrahydroquinoline group.

4. The organometallic compound of any of claims 1-3, wherein
ring CY₁₁ is a benzene group, a pyridine group, a furan group, a thiophene group, or a selenophene group,
X₁ is C(R₁),
X₂ is C(R₂), and
X₃ is C(R₃).

5. The organometallic compound of any of claims 1-4, wherein
ring CY₂₁ is a benzene group; and/or
wherein
R₁₁ is selected from hydrogen, a substituted or unsubstituted C₁-C₆₀ alkyl group, a substituted or unsubstituted C₃-C₁₀ cycloalkyl group, -Si(Q₃)(Q₄)(Q₅), and - Ge(Q₃)(Q₄)(Q₅).

6. The organometallic compound of any of claims 1-5, wherein
R₁₁ is selected from:
hydrogen, deuterium, a methyl group, an ethyl group, an n-propyl group, an iso-propyl group, an n-butyl group, an iso-butyl group, a sec-butyl group, a tert-butyl group, an n-pentyl group, a tert-pentyl group, a neo-pentyl group, an iso-pentyl group, a sec-pentyl group, a 3-pentyl group, a sec-iso-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, a tert-decyl group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, -Si(Q₃)(Q₄)(Q₅), and - Ge(Q₃)(Q₄)(Q₅);
a methyl group, an ethyl group, an n-propyl group, an iso-propyl group, an n-butyl group, an iso-butyl group, a sec-butyl group, a tert-butyl group, an n-pentyl group, a tert-pentyl group, a neo-pentyl group, an iso-pentyl group, a sec-pentyl group, a 3-pentyl group, a sec-iso-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, and a tert-decyl group, each substituted with at least one deuterium; and
a cyclopentyl group, a cyclohexyl group, a cycloheptyl group, a cycloctyl group, an adamantanyl group, a norbornanyl group, a norbornenyl group, a cyclopentenyl group, a cyclohexenyl group, and a cycloheptenyl group, each substituted with at least one selected from deuterium and a C₁-C₁₀ alkyl group.

7. The organometallic compound of any of claims 1-6, wherein
R₂₁ is hydrogen, and
Z₁ and Z₂ are each independently selected from:
a methyl group, an ethyl group, an n-propyl group, an iso-propyl group, an n-butyl group, an iso-butyl group, a sec-butyl group, a tert-butyl group, an n-pentyl group, a tert-pentyl group, a neo-pentyl group, an iso-pentyl group, a sec-pentyl group, a 3-pentyl group, a sec-iso-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, and a tert-decyl group; and
a methyl group, an ethyl group, an n-propyl group, an iso-propyl group, an n-butyl group, an iso-butyl group, a sec-butyl group, a tert-butyl group, an n-pentyl group, a tert-pentyl group, a neo-pentyl group, an iso-pentyl group, a sec-pentyl group, a 3-pentyl group, a sec-iso-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, and a tert-decyl group, each substituted with at least one deuterium;
preferably wherein
Z₁ is selected from:
an iso-propyl group, an iso-butyl group, a sec-butyl group, a tert-butyl group, a tert-pentyl group, a neo-pentyl group, an iso-pentyl group, a sec-pentyl group, a 3-pentyl group, a sec-iso-pentyl group, an iso-hexyl group, a sec-hexyl group, a tert-hexyl group, an iso-heptyl group, a sec-heptyl group, a tert-heptyl group, an iso-octyl group, a sec-octyl group, a tert-octyl group, an iso-nonyl group, a sec-nonyl group, a tert-nonyl group, an iso-decyl group, a sec-decyl group, and a tert-decyl group; and
an iso-propyl group, an iso-butyl group, a sec-butyl group, a tert-butyl group, a tert-pentyl group, a neo-pentyl group, an iso-pentyl group, a sec-pentyl group, a 3-pentyl group, a sec-iso-pentyl group, an iso-hexyl group, a sec-hexyl group, a tert-hexyl group, an iso-heptyl group, a sec-heptyl group, a tert-heptyl group, an iso-octyl group, a sec-octyl group, a tert-octyl group, an iso-nonyl group, a sec-nonyl group, a tert-nonyl group, an iso-decyl group, a sec-decyl group, and a tert-decyl group, each substituted with at least one deuterium, and
Z₂ is selected from a methyl group, an ethyl group, an n-propyl group, an iso-propyl group, an n-butyl group, an iso-butyl group, a sec-butyl group, and a tert-butyl group.

8. The organometallic compound of any of claims 1-7, wherein
the number of carbon atoms included in Z₁ is greater than that of carbon atoms included in Z₂.

9. The organometallic compound of any of claims 1-8, wherein in Formulae 2 and 3, a group represented by 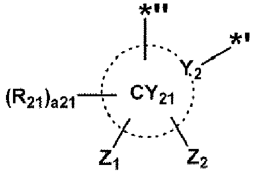 is selected from groups represented by Formulae CY1-1 to CY1-6: 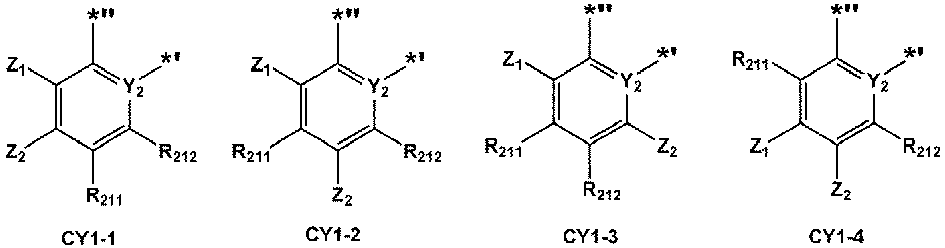 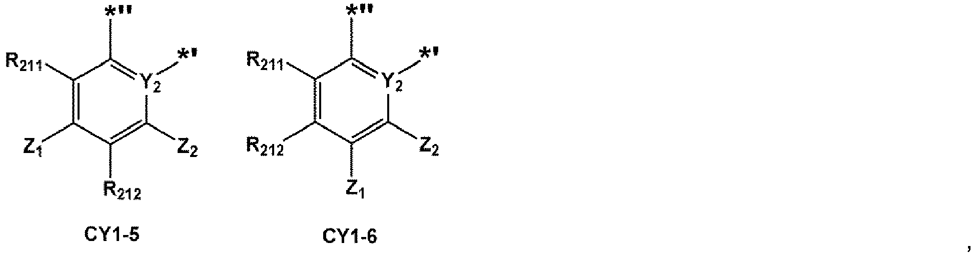 wherein, in Formulae CY1-1 to CY1-6,
R₂₁₁ and R₂₁₂ are each independently defined the same as R₂₁ in claim 1,
Z₁, Z₂, and Y₂ are each independently the same as described in claim 1,
*' indicates a binding site to M in Formula 1, and
*" indicates a binding site to a neighboring group in Formula 2.

10. The organometallic compound of any of claims 1-9, wherein
in Formula 1, L₂ is a bidentate ligand represented by Formula 4: 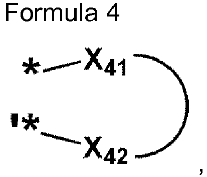 wherein, in Formula 4,
X₄₁ and X₄₂ are each independently O,
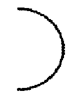 indicates an atomic group linking X₄₁and X₄₂ to each other, and
* and *' each indicate a binding site to M in Formula 1.

11. The organometallic compound of any of claims 1-9, wherein in Formula 1, L₂ is selected from groups represented by Formulae 4A to 4F: 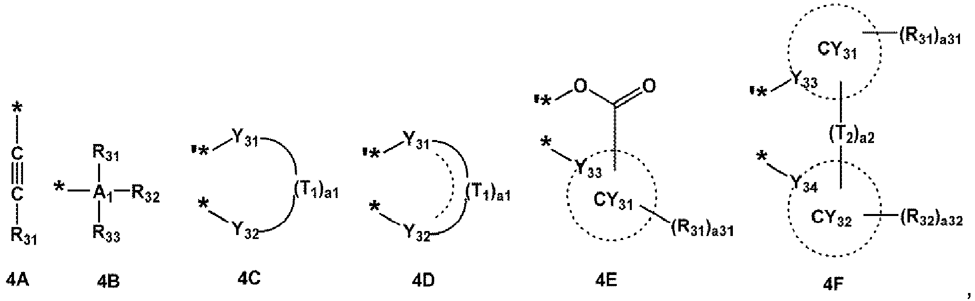 wherein, in Formulae 4A to 4F,
Y₃₁ is selected from O, N, N(R₃₄), P(R₃₄)(R₃₅), and As(R₃₄)(R₃₅),
Y₃₂ is selected from O, N, N(R₃₆), P(R₃₆)(R₃₇), and As(R₃₆)(R₃₇),
T₁ is selected from a single bond, a double bond, *-C(R₃₄)(R₃₅)-*', *-C(R₃₄)=C(R₃₅)-*', *=C(R₃₄)-*', *-C(R₃₄)=*', *=C(R₃₄)-C(R₃₅)=C(R₃₆)-*', *-C(R₃₄)=C(R₃₅)-C(R₃₆)=*', and *-N(R₃₄)-*',
Y₃₃ and Y₃₄ are each independently C or N,
T₂ is selected from a single bond, a double bond, O, S, C(R₃₄)(R₃₅), Si(R₃₄)(R₃₅), or N(R₃₄),
ring CY₃₁ and ring CY₃₂ are each independently selected from a C₅-C₃₀ carbocyclic group and a C₁-C₃₀ heterocyclic group,
A₁ is P or As,
R₃₁ to R₃₇ are each independently defined the same as R₁ in claim 1,
a1 and a2 are each independently an integer from 0 to 10, and
* and *' each indicate a binding site to M in Formula 1;
preferably wherein
in Formula 1, L₂ is a group represented by Formula 4D,
Y₃₁ and Y₃₂ are each independently O,
T₁ is selected from *=C(R₃₄)-C(R₃₅)=C(R₃₆)-*' and *-C(R₃₄)=C(R₃₅)-C(R₃₆)=*',
a1 is1, and
R₃₄ to R₃₆ are each independently selected from:
a methyl group, an ethyl group, an n-propyl group, an iso-propyl group, an n-butyl group, an iso-butyl group, a sec-butyl group, a tert-butyl group, an n-pentyl group, a tert-pentyl group, a neo-pentyl group, an iso-pentyl group, a sec-pentyl group, a 3-pentyl group, a sec-iso-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, and a tert-decyl group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a phenyl group, a biphenyl group, a terphenyl group, a naphthyl group, a pyridinyl group, a pyrimidinyl group, a carbazolyl group, a fluorenyl group, a dibenzosilolyl group, a dibenzofuranyl group, a dibenzothiophenyl group, -Si(Q₃)(Q₄)(Q₅), and -Ge(Q₃)(Q₄)(Q₅);
a methyl group, an ethyl group, an n-propyl group, an iso-propyl group, an n-butyl group, an iso-butyl group, a sec-butyl group, a tert-butyl group, an n-pentyl group, a tert-pentyl group, a neo-pentyl group, an iso-pentyl group, a sec-pentyl group, a 3-pentyl group, a sec-iso-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, and a tert-decyl group, each substituted with at least one deuterium; and
a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a phenyl group, a biphenyl group, a terphenyl group, a naphthyl group, a pyridinyl group, a pyrimidinyl group, a carbazolyl group, a fluorenyl group, a dibenzosilolyl group, a dibenzofuranyl group, and a dibenzothiophenyl group, each substituted with at least one selected from deuterium, a C₁-C₁₀ alkyl group, a phenyl group, and a biphenyl group.

12. The organometallic compound of claim 1, wherein
the organometallic compound is one selected from Compounds 1 to 200:

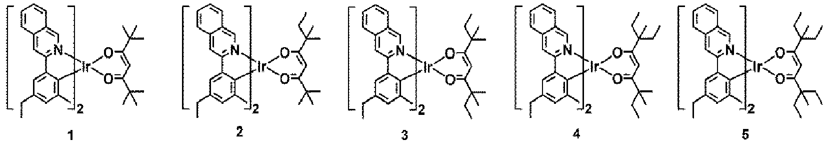

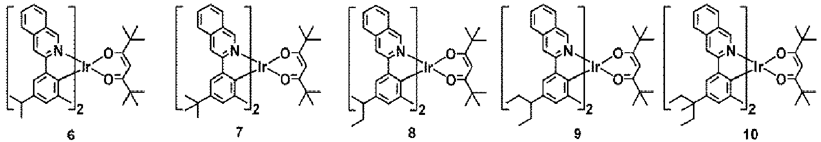

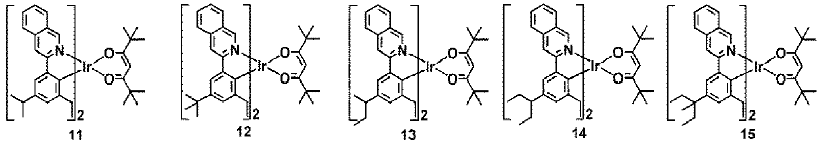

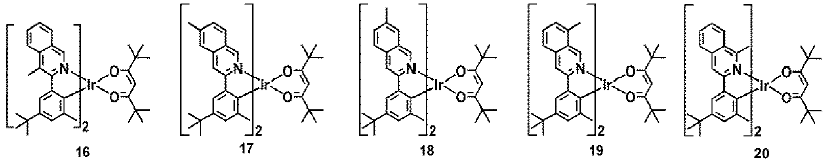

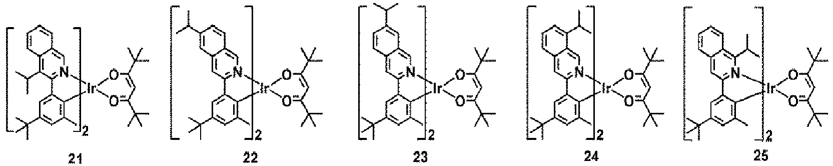

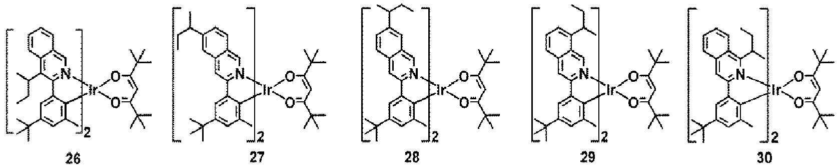

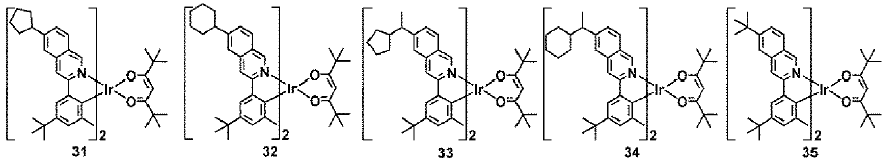

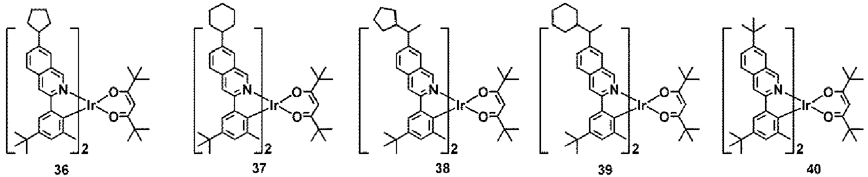

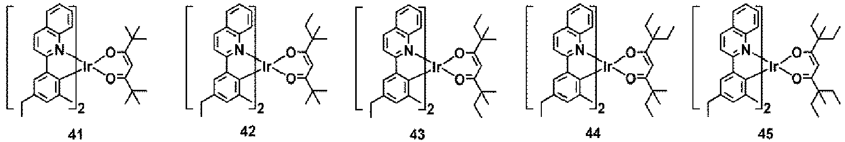

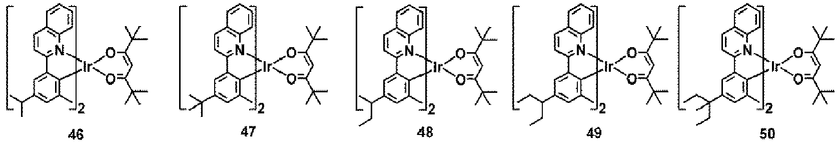

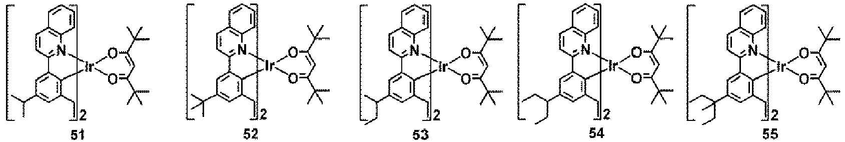

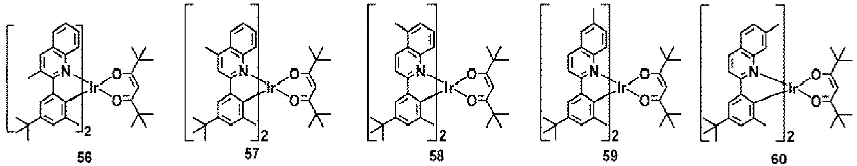

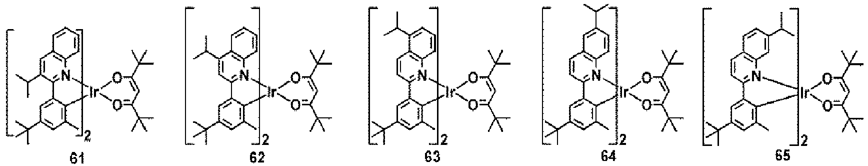

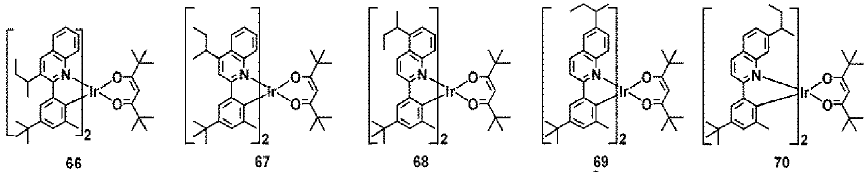

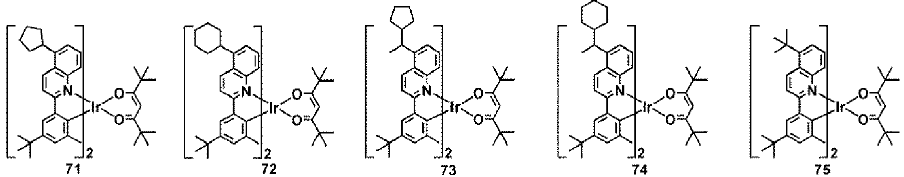

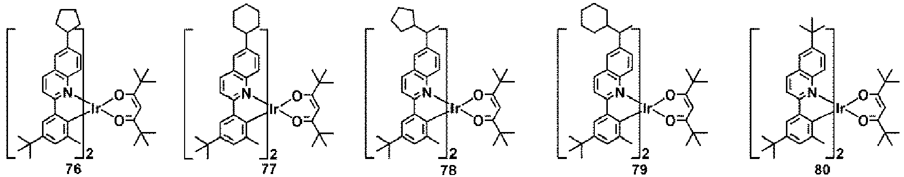

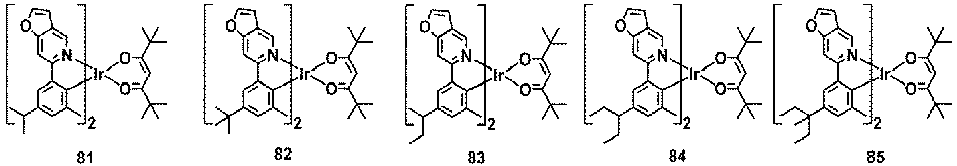

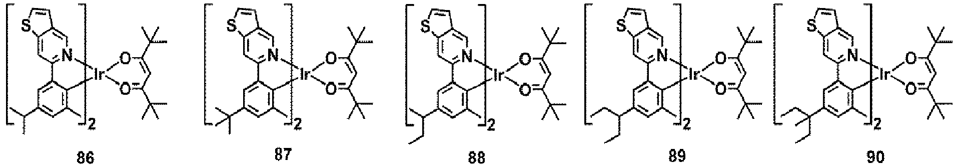

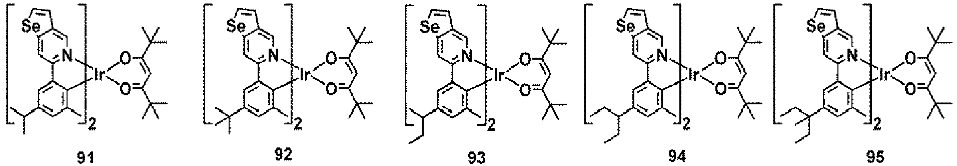

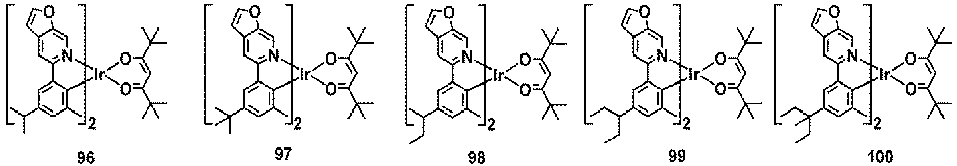

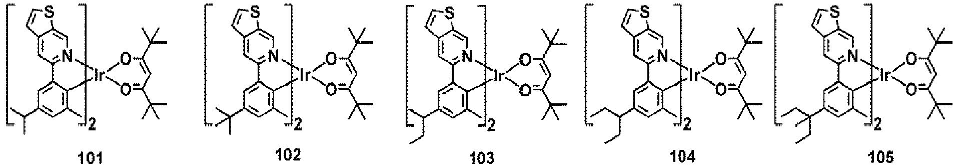

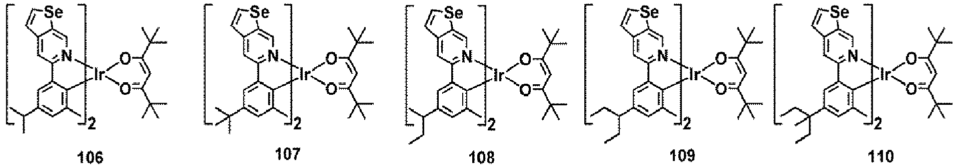

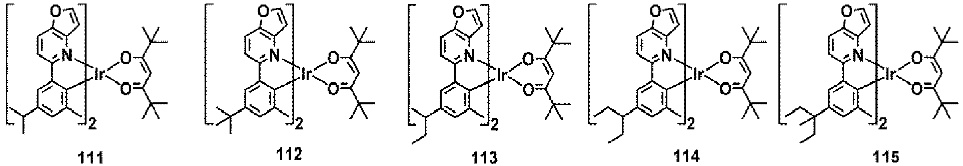

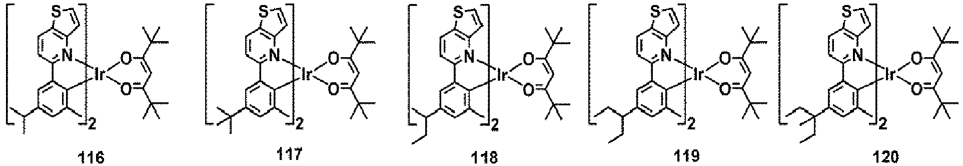

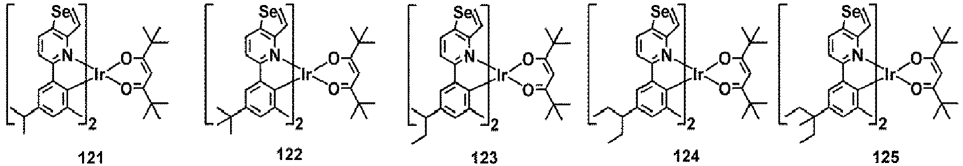

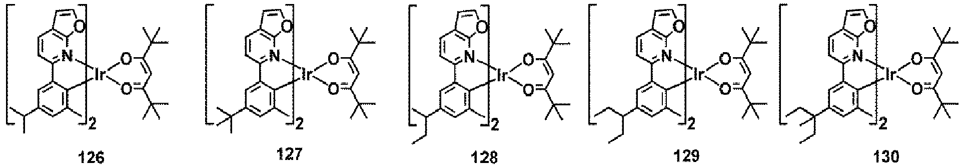

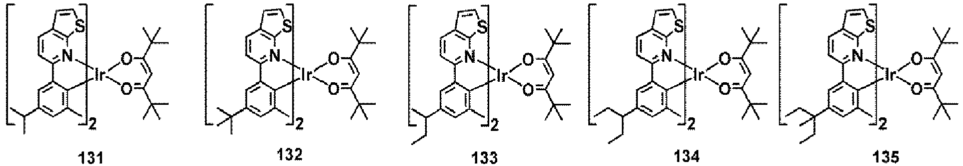

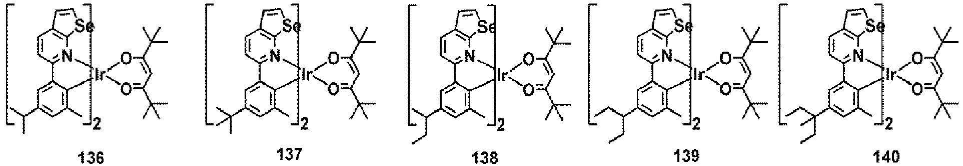

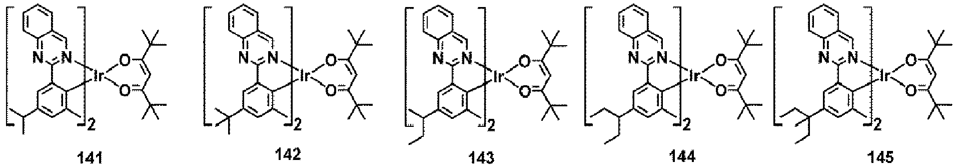

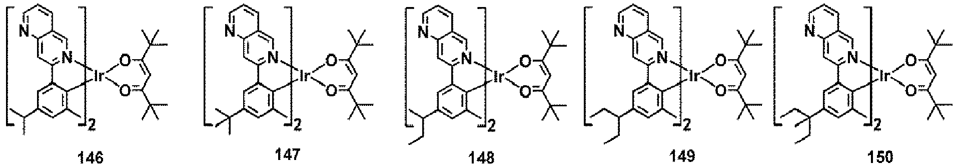

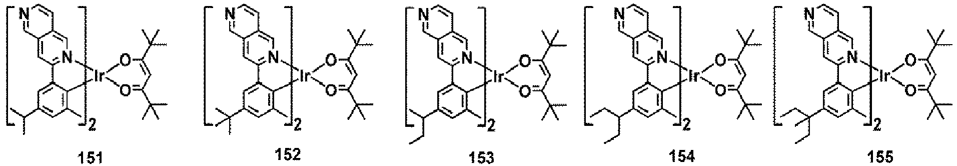

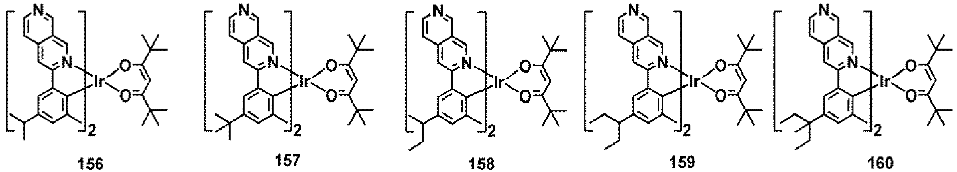

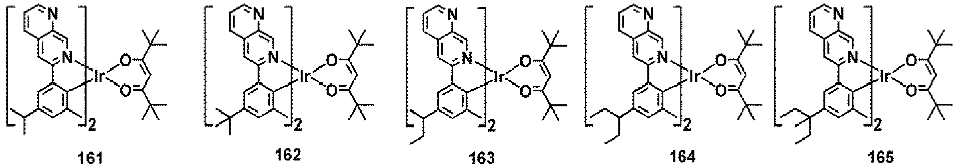

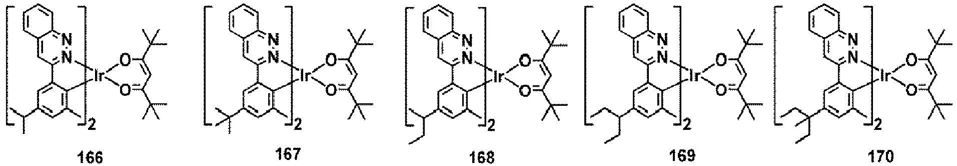

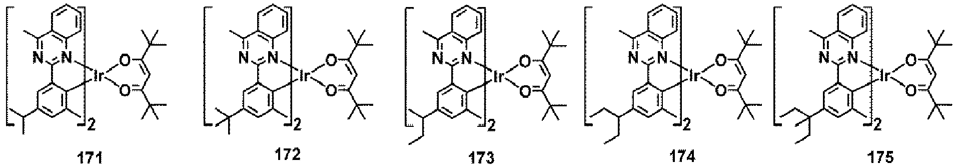

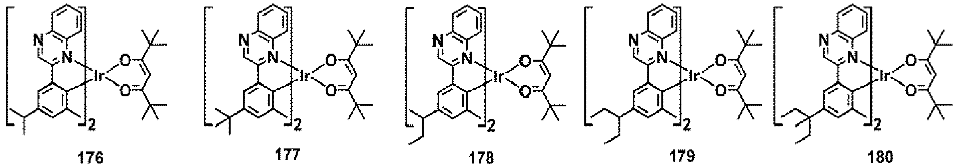

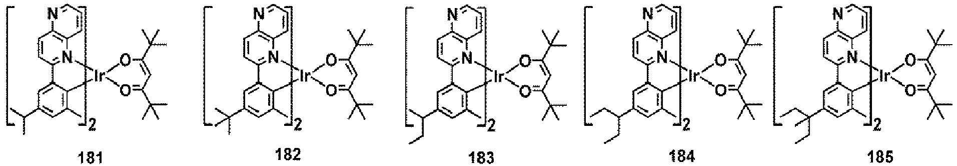

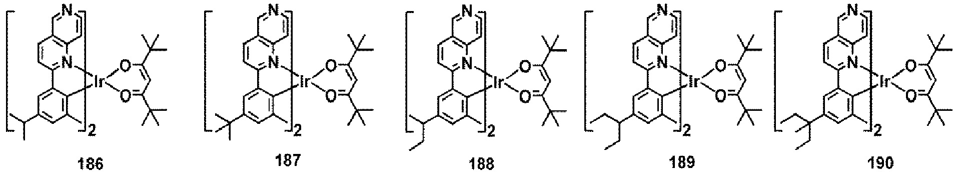

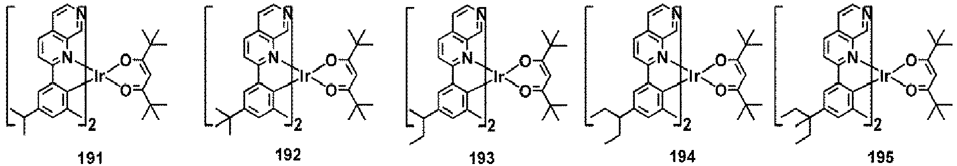

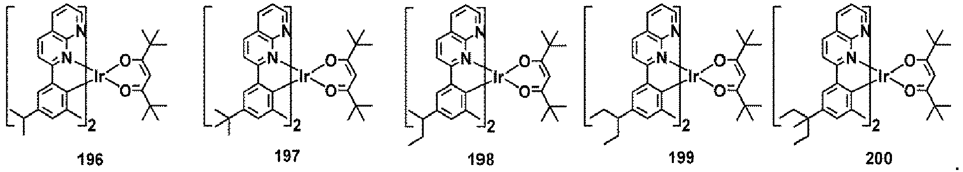

13. An organic light-emitting device comprising:
a first electrode;
a second electrode; and
an organic layer located between the first electrode and the second electrode,
wherein the organic layer comprises an emission layer and at least one of the organometallic compound of any of claims 1-12.

14. The organic light-emitting device of claim 13, wherein
the first electrode is an anode,
the second electrode is a cathode,
the organic layer further comprises a hole transport region located between the first electrode and the emission layer, and an electron transport region located between the emission layer and the second electrode,
wherein the hole transport region comprises a hole injection layer, a hole transport layer, an electron blocking layer, a buffer layer, or any combination thereof, and
wherein the electron transport region comprises a hole blocking layer, an electron transport layer, an electron injection layer, or any combination thereof;
and/or
wherein the emission layer comprises the organometallic compound, preferably wherein the emission layer further comprises a host, wherein an amount of the host in the emission layer is greater than that of the organometallic compound in the emission layer.

15. A diagnostic composition comprising at least one of the organometallic compound of any of claims 1-12.
